# EUROPEAN PATENT APPLICATION

(11) **EP 4 520 318 A1**
(43) Date of publication of application: **12.03.2025**
(21) Application number: 23195719.2
(22) Date of filing: 06.09.2023
(51) Int. Cl.: A61K 9/00, A61K 9/70

(54) **TRANSDERMAL THERAPEUTIC SYSTEM OF LUMATEPERONE**

(71) Applicant: Luye Pharma Switzerland AG, 4051 Basel (CH)
(72) Inventor: WAUER, Gabriel, 83727 Schliersee (DE); HENZEL, Claudia, 80803 Muenchen (DE); SCHURAD, Bjoern, 80807 Muenchen (DE); BENDA, Christian, 83730 Fischbachau (DE); STIEHLE, Andrea, 83714 Miesbach (DE)
(74) Representative: Kraus & Lederer PartGmbB

(57) **Abstract**

The present invention concerns a transdermal therapeutic system for administering the active ingredient lumateperone comprising a backing layer and at least one active ingredient containing layer, comprising the active ingredient in free base or salt form embedded in a polymer matrix, wherein the polymer matrix comprises a polymer selected from the group consisting of (1) a polyacrylate, preferably a polyacrylate containing free hydroxy groups, (2) a mixture of a polyacrylate and a silicone polymer, and (3) one or more silicone polymer. The transdermal therapeutic system may further comprise an adhesive layer, a membrane layer, and a release liner.

## Description

### Technical field

The present invention concerns a transdermal therapeutic system for the administration of lumateperone.

### Background

Lumateperone, i.e. 1 -(4-fluoro-phenyl)-4-((6bR, 10aS)-3-methyl-2,3,6b,9,10,10a-hexahydro-1H,7H-pyrido[3',4':4,5]pyrrolo[1,2,3-de]quinoxalin-8-yl)-butan-1-one, also known as "ITI-007", is a potent 5-HT_{2A} receptor ligand with strong affinity for dopamine (DA)-D2 receptors and the serotonin transporter (SERT). Lumateperone has the following structure: 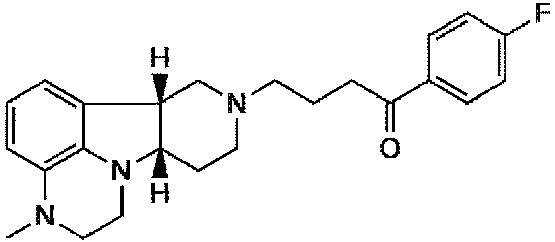 Lumateperone free base

Lumateperone, analogues thereof, salts thereof, and methods for treatment comprising such compounds and methods of manufacturing such compounds, have been disclosed in various patent application documents, including e.g., WO 2020/047241 A1 and the further patent documents mentioned in paragraph [0003] of WO 2020/047241 A1 (in the following referred to as WO'241).

The drug has been approved for schizophrenia in December 2019 and for bipolar depression type I and II in December 2021 in the United States. The commercially available preparation of lumateperone is marketed under the trade name Caplyta^{®}. This product only allows for a once-daily administration in the form of oral capsules with dosages of 10.5 mg, 21 mg or 42 mg lumateperone per capsule. The active ingredient is used in form of its tosylate salt, wherein 60 mg (30 mg or 15 mg) per capsule correspond to 42 mg (21 mg or 10.5 mg) of the lumateperone free base. The oral bioavailability of lumateperone is only about 4.4 %. Moreover, lumateperone has a strong first-pass metabolism in the liver. This makes the provision of alternative administration forms desirable.

Transdermal therapeutic systems (abbreviated herein as: TDS) represent a well-known alternative to other dosage forms, such as oral dosage forms. Transdermal therapeutic systems are often associated with several advantages, including e.g. a higher bioavailability achieved via the transdermal administration, which allows to reduce the drug amount in the formulation; a better patient compliance achieved with a multi-day patch instead of the once daily oral administration; a circumvention of the first-pass; a reduction of plasma level fluctuations.

A transdermal therapeutic system of lumateperone is described in WO'241. The TDS according to WO'241 comprises lumateperone in free base or in salt form, an adhesive polymer, and optionally further excipients including permeation enhancing excipients and antioxidants. Permeation enhancers and certain combinations thereof are used to enhance permeation and the *in vivo* drug delivery. Antioxidants are said to improve the chemical stability of the formulations by preventing oxidative chemical degradation of the active ingredient.

Apart from the permeation properties (achieved with certain permeation enhancers and combinations thereof) and the chemical stability (in the short term), WO'241 fails to address certain further characteristics that are desirable for transdermal therapeutic systems.

While WO'241 generally discloses the use of acrylate adhesives, silicone adhesives and their blends, it does not consider the advantages and disadvantages of using more distinguished polymer adhesives. This in particular relates to absence or presence of certain functional groups in the polymer adhesives, which can have an effect on the adhesiveness, the physical stability, the permeation behaviour, as well as the chemical stability of the system.

For example, WO'241 does not address at all the need for a high adhesiveness. Achieving a high adhesiveness is required for the provision of a patch that can be used for one day and especially important if use is foreseen for several days.

Furthermore, WO'241 does not address the need of providing a physically stable transdermal therapeutic system, i.e. the inhibition of crystallization of the active ingredient during storage. Crystallization of the active ingredient from the TDS is problematic because, besides the adverse effect on therapeutic efficacy and appearance of the matrix, it can also adversely affect in vitro drug release, adhesive strength, tack and shear of the TDS.

While according to WO'241, the drug formulations disclosed therein are chemically stable during storage at room temperature in the short term (i.e. up to two months), the need for chemical stability of the systems at long-term conditions and accelerated conditions over a longer time period (i.e. for several months) has not been addressed in WO'241.

WO'241 moreover does not put any focus on the relation between drug loading and matrix weight. This is an important aspect to be considered for achieving a constant permeation over several days.

Finally, the use of permeation enhancers, which seem to be necessary in the systems according to WO'241 in order to achieve sufficient permeation, are often associated with disadvantages, e.g. that they cause skin irritation. They also complicate the preparation of the transdermal therapeutic system as they are often volatile on drying, sweat out in a lipophilic matrix, and adversely reduce tack and cohesion. From a regulatory point of view, enhancers make the development more difficult and expensive because they must be quantified, controlled, and their impact on the *in vivo* performance must be demonstrated. Specific requirements for enhancers are outlined in the "Guideline on excipients in the dossier for application for marketing authorisation of a medicinal product" (EMEA/CPMP/CVMP/QWP/396951/2006). In view of these disadvantages of using permeation enhancers, it would be desirable to provide a transdermal therapeutic system of lumateperone that has advantageous permeation properties without the need of using an enhancer.

In conclusion, there is still a need for further or improved transdermal therapeutic systems of lumateperone.

It is therefore the object of the present invention to provide a physically and chemically stable transdermal therapeutic systems of lumateperone showing a sufficiently high and substantially constant permeation as well as a high adhesiveness over preferably several days, such as e.g. at least 2 or 3 days, which allows the use of the transdermal therapeutic system as a multiday patch. Ideally, such transdermal therapeutic systems do not require the use of a permeation enhancer.

### Brief Summary

The present invention attempts to solve this object by the following aspects.

In a first aspect, the present invention provides a transdermal therapeutic system for administering the active ingredient lumateperone through the skin of a patient comprising the following layers:
a) a backing layer and
b) at least one active ingredient containing layer, comprising the active ingredient in free base or salt form embedded in a polymer matrix,
**characterized in that**
the polymer matrix comprises a polymer selected from the group consisting of (1) a polyacrylate, preferably a polyacrylate containing free hydroxy groups, (2) a mixture of an acrylate polymer and/or copolymer and a silicone polymer, and (3) a silicone polymer.

It has been found that the transdermal therapeutic systems according to the present invention have a sufficiently high and substantially constant permeation, preferably without the use of a permeation enhancer.

A "constant permeation" as used herein means that the permeation rate, expressed in amount permeated per unit of time and unit surface area, is not significantly changed over a prolonged time interval so that the cumulative permeation profile has a substantially linear course. However, in particular in the initial phase, a so-called period of lag time can occur, wherein the permeation has not the constant permeation rate. Moreover, the permeation rate may be reduced at very late time points due to a very strong discharge of the system. The permeation can be determined by permeation tests *per* se known to the skilled person, for example by the *in vitro* permeation test according to NovoCell Schönbach according to OECD (2004) Test Guideline 428 "Skin absorption: In vitro Method & Series on testing and assessment", No. 28 "Guidance document for the conduct of skin absorption studies".

Moreover, it has been found that the transdermal therapeutic systems according to the present invention have a high adhesiveness suitable for at least one day, such as e.g. 2 or 3 days, which allows the use of the transdermal therapeutic systems as a multiday patch.

Moreover, the transdermal therapeutic systems of the present invention are physically and chemically stable during storage.

In a second aspect, the present invention provides a transdermal therapeutic system in accordance with the first aspect, wherein the system further comprises
c) an adhesive layer on the active ingredient containing layer,
wherein the adhesive layer comprises at least one adhesive polymer,
wherein the adhesive layer is initially free of the active ingredient, and
wherein the adhesive layer is the skin-contacting layer of the system.

"Initially free of active ingredient" means that the adhesive layer does not contain any active ingredient before coming into contact with the active ingredient layer. Once laminated, a certain amount of active ingredient diffuses from the active-ingredient layer into the adhesive layer.

It has been found that by using an adhesive layer in the transdermal therapeutics system in accordance with the second aspect of the present invention, the time period wherein permeation is constant can be increased, e.g. by 1 or 2 days, e.g. from 2 days to 3 days. Moreover, the adhesiveness of the transdermal therapeutic system can be increased. This prolongs the time period the transdermal therapeutic system can be used compared to a system which does not comprise an adhesive layer, e.g. by 1 or 2 days.

In a third aspect, the present invention provides a transdermal therapeutic system in accordance with the second aspect, wherein the system further comprises
d) a membrane layer between the active ingredient containing layer and the adhesive layer,
wherein the membrane layer comprises a membrane polymer that is permeable for the active ingredient and controls the release of the active ingredient.

It has been found that by using a membrane layer between the active ingredient containing layer and the adhesive layer in accordance with the third aspect of the present invention, the time period, wherein permeation is constant, can be increased, e.g. by 1 day to up to 4 days, compared to a system, which does not comprise a membrane layer.

In a further aspect, the present invention provides a method for preparing a transdermal therapeutic system in accordance with the present invention, according to any one of the preceding claims, comprising the following steps:
(a) preparing an active ingredient containing layer composition,
(b) coating the composition prepared in step (a) onto a release liner (i),
(c) drying the component prepared in step (b), and
(d) laminating a backing layer onto the active ingredient containing layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa*;
or optionally comprising the following steps in addition to steps (a)-(d):
(j) laminating a membrane layer suitable to control the release of the active ingredient onto the active ingredient containing layer of the component prepared in accordance with step (h),
(k) performing steps (e) to (g),
(I) laminating the adhesive layer of the component prepared in step (k) onto the membrane layer of the component prepared in step (j), or *vice versa.*

In a further aspect, the present invention relates to the use of a polymer selected from the group consisting of (1) a polyacrylate, preferably a polyacrylate containing free hydroxy groups, (2) a mixture of a polyacrylate and a silicone polymer, and (3) a silicone polymer for stabilizing lumateperone free base in a transdermal therapeutic system or reducing degradation of lumateperone free base in a transdermal therapeutic system, preferably the transdermal therapeutic system of the present invention.

In a further aspect, the present invention relates to the used of an antioxidant, preferably ascorbic acid or tocopherol, in the transdermal therapeutic system of the present invention for reducing, preferably inhibiting the formation of N-nitrosamines in the system.

In a further aspect, the transdermal therapeutic system of the present invention is provided for use in a method for treating a disease selected from the group consisting of major depressive disorder (MDD) and adjunctive mixed features associated therewith, bipolar depression I or II and schizophrenia.

In a further aspect of the present invention, lumateperone is provided for use in a method for treating a disease selected from the group consisting of major depressive disorder (MDD) and adjunctive mixed features associated therewith, bipolar depression I or II and schizophrenia, wherein lumateperone is administered in free base or salt form through the skin of a patient by means of the transdermal therapeutic system of the present invention.

The above-mentioned various aspects are to be understood that they can be freely combined with each other unless otherwise indicated.

### Description of the Figures

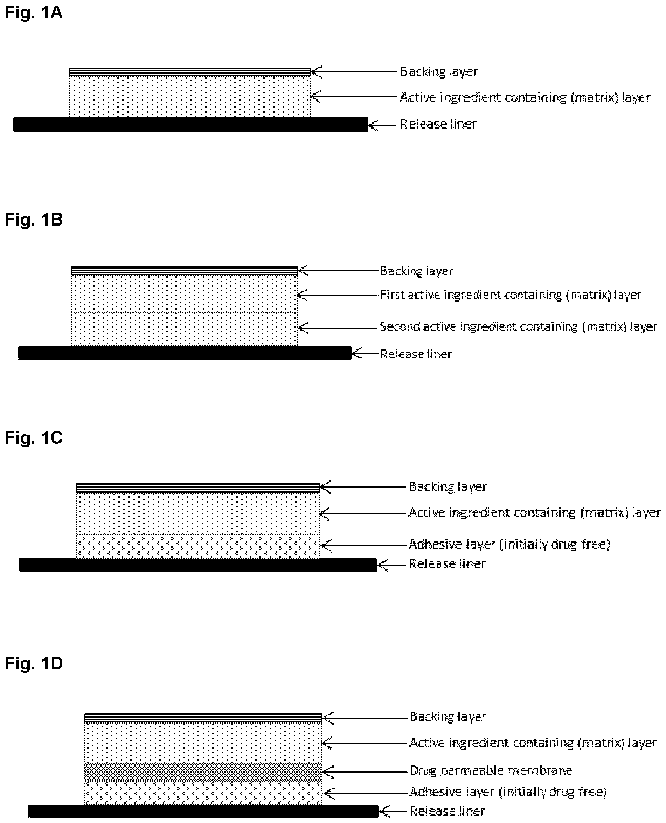

**Fig. 1** is a cross-sectional view of the TDS according to the present invention according to its various aspects. The TDS according to **Fig. 1A** comprises a backing layer, and active ingredient containing layer, and a release liner in accordance with the first aspect of the invention. The TDS according to **Fig. 1B** comprises a backing layer, a first active ingredient containing layer, a second active ingredient containing layer, and a release liner, also in accordance with the first aspect of the invention. The TDS according to **Fig. 1C** comprises a backing layer, an active ingredient containing layer, an adhesive layer, and a release liner in accordance with the second aspect of the invention. The TDS according to **Fig. 1D** comprises a backing layer, an active ingredient containing layer, a membrane layer, an adhesive layer, and a release liner in accordance with the third aspect of the invention.
**Fig. 2** shows the adhesive strength measured for various samples prepared in Example 1 after storage at 40°C/75% RH compared to prior art patches Neupro^{®} (1 day patch comprising rotigotine base and BIO-PSA Q7-4301 & 4201), FNT (3 days patch comprising fentanyl base and Duro-Tak^{®} 87-4098) and FTA (3 days patch comprising fentanyl base and Duro-Tak^{®} 387-2510).
**Fig. 3** shows the tack for various samples prepared in Example 1 measured after storage at 40°C/75% RH compared to prior art patch Neupro^{®}.
**Fig. 4** shows the separation force needed to remove the release liner, measured for various samples prepared in Example 1 after storage at 40°C/75% RH compared to prior art patch FTA.
**Fig. 5** shows the cumulatively permeated amount of lumateperone [µg/cm²] for various samples prepared in Example 1 on human skin model (HSE) over a period 96 hours.
**Fig. 6** shows the cumulatively permeated amount of lumateperone [µg/cm²] for various samples prepared in Example 1 on human skin model (HSE) over a period of 96 hours.
**Fig. 7** shows the adhesive strength measured for various samples prepared in Example 2 after storage at 40°C/75% RH compared to prior art patches Neupro^{®}, FNT, and FTA.
**Fig. 8** shows the tack for various samples prepared in Example 2 measured after storage at 40°C/75% RH compared to prior art patch FTA.
**Fig. 9** shows the separation force needed to remove the release liner, measured for various samples prepared in Example 2 after storage at 40°C/75% RH compared to prior art patch FTA.
**Fig. 10** shows the cumulatively permeated amount of lumateperone [µg/cm²] for various samples prepared in Example 2 on human skin model (HSE) over a period of 96 hours.
**Fig. 11** shows the cumulatively permeated amount of lumateperone [µg/cm²] for various samples prepared in Example 3 on human skin model (HSE) over a period of 96 hours.
**Fig. 12** shows the cumulatively permeated amount of lumateperone [µg/cm²] for a sample prepared in Example 4 on human skin model (HSE) over a period of 96 hours.
**Fig. 13** shows the sum of degradation products from lumateperone of samples prepared in Example 1 after storage at 40°C/75% RH for 6 months.

### Detailed Description

In the following, the present invention will be described in further detail and, unless it is explicitly stated otherwise, the explanations on the individual features refer to all of the aspects of the present invention and can be freely combined with one another.

### Definitions

The transdermal therapeutic system ("TDS") of the present invention can also be designated as a patch. The TDS of the present invention is for application on the skin of a patient in need thereof, i.e. a patient in need of lumateperone treatment.

The "active ingredient" within the meaning of the present invention is lumateperone, i.e. 1-(4-fluoro-phenyl)-4-((6bR,10aS)-3-methyl-2,3,6b,9,10,10a-hexahydro-1H,7H-pyrido[3',4':4,5]pyrrolo[1 ,2,3-de]quinoxalin-8-yl)-butan-1-one. The active ingredient is used in its free base form or in salt form in the TDS of the present invention. "In salt form" means in pharmaceutically acceptable salt form, wherein the salt form can be amorphous or crystalline polymorph. The salt form of lumateperone is selected from the group consisting of a tosylate, oxalate, cyclamate, 4-aminosilicylate, or hydrochloride salt form, with the tosylate being the preferred salt form. However, in the present invention, it is preferred that the free base form of lumateperone is used. This applies to all aspects and embodiments of the present invention.

A "polymer matrix" is a solid or semi-solid composition having a three-dimensional structure which comprises a polymer or a mixture of polymers. In the present invention, the polymer matrix comprises an acrylate polymer or copolymer containing free hydroxy groups. The polymer matrix is also referred to as polymer skeleton since the three-dimensional skeleton structure is as a rule provided by the polymer or mixture of polymers. Preferably, the active ingredient is evenly distributed in the polymer matrix.

As described herein, the term "a" refers to one or more elements. For example, the term "an acrylate polymer" refers to one or more acrylate polymers.

Percentages, unless otherwise indicated, refer to weight percentages, abbreviated "wt.-% ". Weight percentage amounts, unless otherwise indicated, are given in relation to a dried product, for example a dried active ingredient containing layer or a dried adhesive layer.

In the TDS according to the invention, the active ingredient is chemically sufficiently stable. "Chemically sufficiently stable" means that degradation products of the active ingredient after 1 month of storage at 40°C and 75% relative air humidity (RH) in total are preferably not more than about 1% by weight, more preferably not more than about 0.5% by weight, based on the desired content of active ingredient in the system. Preferably, the total content of the degradation products after 3 months of storage at 40°C and 75% relative air humidity is less than about 2% by weight, more preferably less than about 0.6% by weight. It is also preferred that the total content of the degradation products after 6 months of storage at 40°C and 75% relative air humidity is less than about 4% by weight, more preferably less than 3% by weight, even more preferably less than 2% by weight. The information on "wt.-%" of degradation products always refer to the amount of active ingredient in the formulation, unless stated otherwise.

In the TDS according to the present invention, the active ingredient is also physically stable. "Physically stable" means that crystallization of the active ingredient is inhibited during storage at room temperature for at least 12 months, or at 40°C and 75% relative air humidity for at least 6 months, preferably 12 months. "Physically stable" also means that the formation of a biphasic system is preferably inhibited under these conditions.

Adhesive strength, tack and separation force of transdermal therapeutic systems of the present invention preferably also do not relevantly change during storage.

The application period of a TDS according to the invention is at least one day, preferably at least 2 days, such as e.g. 2 to 3 days, more preferably at least 3 days, such as e.g. 3 to 4 days.

Although it is possible to use a permeation enhancer in the TDS of the present invention (i.e. in the active ingredient containing layer and/or the adhesive layer), it is preferred that the TDS of the present invention does not comprise a permeation enhancer. In particular, the TDS of the present invention does not comprise the permeation enhancers used in WO'241, which include fatty acid esters (e.g. lauryl lactate, isopropyl myristate, oleyl oleate, methyl laurate, isopropyl palmitate, ethyl oleate), fatty alcohols (dodecanol, octyldecanol, lauryl alcohol, alcohols (e.g. propylene glycol), amine oxides (e.g. dimethyldodecyl amine oxide, myristamine oxide), carboxylic acids (e.g. an alpha-hydroxy acid, e.g. lactic acid), caprates (e.g. methyl caprate, propylene glycol dicaprate/dicaprylate), and combinations thereof such as e.g. lauryl lactate/propylene glycol.

### Structure of the TDS

The TDS of the present invention comprises several layers.

The backing layer is situated on the back side of the TDS.

The active ingredient containing layer is situated onto the side of the backing layer which is in direction to the skin after TDS application. In the first aspect of the present invention, the active ingredient containing layer is the skin-contacting layer, which is protected by a release liner during storage. The first aspect of the present invention thus concerns a TDS comprising the following layers in the following order (cf. **Fig. 1A**):
1. a backing layer
2. a first active ingredient containing layer
3. a release liner.

In one embodiment, the TDS of the present invention comprises two active ingredient containing layers laminated to each other. In that case, a TDS in accordance with the first aspect of the present invention comprising the following layers in the following order (**Fig. 1B**).
1. a backing layer
2. a first active ingredient containing layer
3. a second active ingredient containing layer
4. a release liner.

In the second aspect of the present invention, an adhesive layer is deposited to the active ingredient containing layer that faces the human skin in use. In this second aspect of the present invention, the adhesive layer is the skin-contacting layer, which is protected by a release liner during storage. The second aspect of the present invention thus concerns a TDS comprising the following layers in the following order (**Fig. 1C**).
1. a backing layer
2. an active ingredient containing layer
3. an adhesive layer
4. a release liner.

In the third aspect of the present invention, the TDS comprises a membrane layer deposited between the active ingredient containing layer and the adhesive layer. The third aspect of the present invention thus concerns a TDS comprising the following layers in the following order (**Fig. 1D**).
1. a backing layer
2. an active ingredient containing layer
3. a membrane layer
4. an adhesive layer
5. a release liner

Before the TDS is used, the release liner is deposited to the active ingredient containing layer (first aspect) and the adhesive layer (second and third aspect of the invention), respectively, and protects those layers before use of the TDS. The release liner is removed right before the use of the TDS.

In one embodiment, the area of the backing layer of the TDS according to the invention corresponds at least to the area of the active ingredient containing layer or the adhesive layer, respectively.

### Active ingredient containing layer

The TDS according to the present invention comprises at least one active ingredient containing layer. Typically, this means that the TDS of the present invention contains one active ingredient containing layer. However, it is also possible that the TDS of the present invention contains two active ingredient containing layers, in particular if the weight of the active ingredient containing layer exceeds 100 g/m². In that case, two active ingredient containing layers may be used instead of one. The two layers are typically identical in terms of their composition and size and are laminated to one another to achieve a weight of 100 g/m² and more, such as e.g. 100 to 150 g/m². For example, if a matrix weight of 130 g/m² is attempted, two active ingredient containing layers with a matrix weight of 75 g/m² each can be used.

The active ingredient containing layer of the TDS according to the invention contains the active ingredient lumateperone in free base form or in salt form, embedded in a polymer matrix.

Preferably, the active ingredient containing layer of the TDS according to the present invention contains 5 to 20 wt.-%, more preferably 10 to 15 wt.-%, particularly preferably 15 wt.-% of the active ingredient lumateperone.

Preferably, the active ingredient containing layer of the TDS according to the present invention contains 65 to 95 wt.-%, more preferably 70 to 90 wt.-% or 75 to 85 wt.-%, particularly preferably 80 to 85 wt.-% of the polymer matrix.

The polymer matrix comprises a polymer selected from the group consisting of (1) a polyacrylate, (2) a mixture of a polyacrylate and a silicone polymer, and (3) one or more silicone polymer.

The (1) polyacrylate is preferably a polyacrylate containing free hydroxy groups, which are also preferred as matrix polymers over (2) a mixture of a polyacrylate and a silicone polymer and (3) a silicone polymer as matrix polymers.

### (1) Polyacrylates

The polyacrylates suitable for use as matrix polymer in the present invention include homopolymers, copolymers and block-copolymers.

Polyacrylates with or without functionalization can be used in the present invention, that is polyacrylates that are substantially free of free functional groups, and polyacrylates that contain free hydroxy groups and/or free carboxylic acid groups as functional groups.

"Polyacrylates that are substantially free of free functional groups" in the context of the present invention means polyacrylates that are substantially free of hydroxy groups, carboxylic acid groups and amine groups. "Substantially free" in this context means that the total proportion of monomers containing free hydroxy groups, free carboxylic acid groups or free amine groups is below 1% by weight, preferably below 0.5% by weight, more preferably below 0.2% by weight, based on the mixture of monomers the polyacrylate is prepared of. In a particular embodiment, the total proportion of said monomers is below 0.1% by weight. In another particular embodiment, no free hydroxy groups and no free carboxylic acid groups, and no free amine groups are contained in the mixture of monomers.

Polyacrylates substantially free of free functional groups suitable for use in the present invention are typically on the basis of acrylic acid esters and/or methacrylic acid esters.

These polyacrylates can be prepared by the polymerization of acrylic acid esters and/or methacrylic acid esters, with the acrylic acid esters and/or methacrylic acid esters being preferably selected from the group consisting of n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethylhexyl methacrylate, isobornyl methacrylate, isobutyl methacrylate, isopropyl acrylate, isopropyl methacrylate, and mixtures thereof.

These polyacrylates can also be prepared by the copolymerization of acrylic acid esters and/or methacrylic acid esters with vinyl acetate, wherein the acrylic acid esters and/or methacrylic acid esters are selected from the group consisting of n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethyhexyl methacrylate, isobornyl methacrylate, isobutyl methacrylate, isopropyl acrylate, isopropyl methacrylate, and mixtures thereof.

These monomers are esters of the acrylic or methacrylic acid, respectively, that carry linear, branched or cyclic aliphatic C1-C12 substituents without other free functional groups.

For example, a preferred copolymer containing substantially no free functional groups is an acrylate-vinyl acetate copolymer which can be prepared from the starting monomers 2-ethylhexyl acrylate and vinyl acetate, e.g. an acrylate-vinyl acetate copolymer prepared from 50% each of the starting monomers 2-ethylhexyl acrylate and vinyl acetate (Duro-Tak^{®} 87-4098).

The polyacrylates of the present invention may also contain free functional groups. In the context of the present invention, the polyacrylates may contain free hydroxy groups and/or free carboxylic acid groups as free functional groups. The polyacrylates of the present invention however do not substantially contain free amine groups.

Polyacrylates containing free functional groups can be produced from monomers, such as acrylic acid, methacrylic acid, and esters of the acrylic acid or methacrylic acid containing free functional groups.

The proportion of monomers containing free functional groups used in the preparation of polyacrylates containing free functional groups suitable for use in the present invention (i.e. polyacrylates containing free hydroxy groups and/or free carboxylic acid groups as free functional groups, preferably polyacrylates containing free hydroxy groups) is typically at least 1% by weight, preferably at least 2% by weight, more preferably at least 5% by weight based on the mixture of monomers the polyacrylate is prepared of. Typically, the proportion does not exceed 30% by weight and is preferably not more than 20% by weight, more preferably not more than 10% by weight, based on the mixture of monomers the polyacrylate is prepared of.

For the production of polyacrylates containing free carboxylic acid groups, acrylic acids and methacrylic acids or esters of the acrylic acid or methacrylic acid that contain free carboxylic acid groups can be used.

Examples for polyacrylates containing free carboxylic groups suitable for use in the present invention include acrylate-vinyl acetate copolymers containing free carboxyl groups, such as the commercial products Duro-Tak^{®} 387-2051/87-2051 and Duro-Tak^{®} 387-2054/87-2054.

Preferably, however, the polyacrylates used in the present invention do not substantially contain free carboxylic acid groups.

Polyacrylates containing free hydroxy groups are preferred over polyacrylates containing free carboxylic acid groups as well as over polyacrylates which are substantially free of free functional groups.

Polyacrylates containing free hydroxy groups can be prepared by the polymerization of acrylic acid esters and/or methacrylic acid esters containing free hydroxy groups, with the acrylic acid esters and/or methacrylic acid esters being preferably selected from the group consisting of n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethylhexyl methacrylate, isobornyl methacrylate, isobutyl methacrylate, isopropyl acrylate, isopropyl methacrylate, and mixtures thereof, wherein these monomers are substituted with free hydroxy groups.

Polyacrylates containing free hydroxy groups can also be prepared by the copolymerization of acrylic acid esters and/or methacrylic acid esters containing free hydroxy groups with vinyl acetate, wherein the acrylic acid esters and/or methacrylic acid esters are selected from the group consisting of n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethyhexyl methacrylate, isobornyl methacrylate, isobutyl methacrylate, isopropyl acrylate, isopropyl methacrylate, and mixtures thereof, wherein these monomers are substituted with free hydroxy groups.

Examples for acrylate polymers containing free hydroxy groups include the following commercial products: Duro-Tak^{®} 87-202A (with added crosslinker), Duro-Tak^{®} 387-2510, Duro-Tak^{®} 87-2510. Examples for acrylate copolymers containing free hydroxy groups include the following commercial products: Duro-Tak^{®} 387-2516, Duro-Tak^{®} 87-2516, Duro-Tak^{®} 87-20A, Duro-Tak^{®} 387-2287, Duro-Tak^{®} 87-2287, Duro-Tak^{®} 87-4287, Duro-Tak^{®} 387-2525 (with added crosslinker), Duro-Tak^{®} 87-2525 (with added crosslinker).

Particularly preferred polyacrylates for use in the polymer matrix are acrylate-vinyl acetate copolymers containing free hydroxy groups, especially the products Duro-Tak^{®} 387-2516 and 87-2516, which is poly[(2-ethylhexyl)methacrylate-co-vinylacetate-co-(2-hydroxyethyl)methacrylate-co-(2,3-epoxypropyl)methacrylate], wherein the ratio of components is typically about 67 : 28 : 5 : 0.15.

Preferably, the polymer matrix does not comprise acrylate polymers and/or acrylate copolymers other than acrylate polymers and/or acrylate copolymers containing free hydroxy groups.

It has been found in the present invention that when using a polymer matrix based on acrylate polymers and/or acrylate copolymers containing free hydroxy groups, the permeation can be further increased compared to acrylate polymers or copolymers with substantially no functional groups, is achieved. Moreover, it has been found that acrylate polymers and/or acrylate copolymers containing free hydroxy groups have an additional stabilizing effect on the active ingredient lumateperone free base contained in the active ingredient containing layer compared to acrylate polymers or copolymers with substantially no functional groups. That is, the chemical stability of lumateperone free base in the TDS can be further improved by using an acrylate polymer and/or copolymer containing free hydroxy groups compared to a polyacrylate with substantially no functional groups, and less degradation products are formed during storage.

It has further been found in the present invention that when using a polymer matrix based on acrylate polymers and/or acrylate copolymers with free hydroxy groups, the permeation can significantly be further increased compared to acrylate polymers or copolymers containing free carboxy groups is achieved. Moreover, it has been found that acrylate polymers and/or acrylate copolymers with free hydroxy groups have an additional stabilizing effect on the active ingredient lumateperone free base contained in the active ingredient containing layer compared to acrylate polymers or copolymers containing free carboxy groups. That is, the chemical stability of lumateperone free base in the TDS can be further improved by using an acrylate polymer and/or copolymer containing free hydroxy groups compared to a polyacrylate containing free carboxy groups, and significantly less degradation products are formed during storage.

### (2) Mixture of a polyacrylate and a silicone polymer

A mixture of a a polyacrylate and a silicone polymer is also suitable as polymer for the polymer matrix, however, it is less preferred than a polyacrylate-silicone mixture and acrylate polymers and/or copolymers with free hydroxy groups as matrix polymers.

The polyacrylate suitable for use in this mixture are as described above. Preferably a polyacrylate containing free hydroxy groups as described above is used.

Examples of suitable silicone adhesives include BIO-PSA 7-4302 (high tack) and BIO-BIO-PSA 7-4202 (medium tack), as well as BIO-PSA SRS7-4602 (high tack) and BIO-PSA SRS7-4502 (medium tack).

The ratio of acrylate polymer and silicone polymer is preferably 1:1 or 1:2.

### (3) Silicone polymer

Silicone polymers can be used as polymer for the polymer matrix in the active ingredient containing layer of the present invention, however, silicone polymers are less preferred than a polyacrylate-silicone mixture and acrylate polymers and/or copolymers with free hydroxy groups as matrix polymers.

A single silicone polymer can be used, however, it is preferred to use a mixture of silicone polymers, in particular a mixture of a high tack and a medium tack silicone adhesive. The ratio of high tack silicone adhesive and medium tack silicone adhesive is preferably 7:3 or 6:4.

Examples of suitable silicone adhesives include BIO-PSA 7-4302 (high tack) and BIO-BIO-PSA 7-4202 (medium tack), as well as BIO-PSA SRS7-4602 (high tack) and BIO-PSA SRS7-4502 (medium tack).

Preferably, silicone polymers are used, which are provided in ethyl acetate as solvent. These are advantageous in the production of the TDS of the present invention because lumateperone free base is soluble therein.

In one embodiment, the active ingredient containing layer consists of active ingredient and polymer matrix.

Optionally, further excipients known in the prior art are contained in the active ingredient containing layer besides the active ingredient and the matrix polymer. Preferably, such further excipients are selected from the group consisting of antioxidants, permeation enhancers, plasticizers, tackifiers, crystallization inhibitors and cohesion increasing substances. Further, substances may be added that trigger the *in situ* release of lumateperone free base in case the active ingredient is used in a salt form.

The excipients are typically used in an amount of up to 15 wt.-% in the active ingredient containing layer, such as e.g. 0.05 to 15 wt.-%, 0.1 to 15 wt.-%, 0.5 to 15 wt.-%, 1 to 15 wt.-%, 1 to 10 wt.-%, 1 to 5 wt.-%, 1 to 2.5 wt.-%, 2 to 5 wt.-%, 2.5 to 5 wt.-%, 2.5 to 10 wt.-%, 5 to 10 wt.-%, or 10 to 15 wt.-%, based on the overall weight of the (dried) active ingredient containing layer.

Suitable antioxidants for use in the active ingredient containing layer of the system of the present invention include tocopherol, butylated hydroxytoluene (BHT), propyl gallate (OPG), N,N'-bis[3-propanamid] Irganox^{®}, ris(2,4-di-tert-butylphenyl) phosphite Irgafos^{®}, ascorbic acid, ascorbyl palmitate, and combinations thereof. The antioxidant is typically contained in the active ingredient containing layer in an amount of 0.05 to 2 wt.-%, preferably 0.05 to 1 wt.-%, more preferably 0.05 to 0.5 wt.-%, even more preferably 0.05 to 0.1 wt.-%, based on the overall weight of the active ingredient containing layer. Tocopherol or/and ascorbyl palmitate are preferred antioxidants to be used in the active ingredient containing layer. Antioxidants have the effect that they improve the chemical stability of the active ingredient in particular during storage of the TDS by preventing oxidative chemical degradation.

It has also been found by the present inventors that the use of an antioxidant in the TDS of the present invention, in particular in the active ingredient containing layer of the TDS of the present invention, advantageously reduces, preferably inhibits the formation of N-nitrosamines in particular during storage of the transdermal therapeutic system due to degradation of the active ingredient. The skilled person understands the term N-nitrosamines and what compounds these are. N-nitrosamines are carcinogenic compounds that are accepted by regulatory authorities only in extremely small amounts in pharmaceutical products including transdermal therapeutic systems. N-nitrosamines may form in active ingredients that have a secondary or tertiary amine function (such as lumateperone) under certain conditions that promote formation of nitrosamines (e.g. high temperatures during storage in the finished product). It has been found by the present inventors that antioxidants, in particular ascorbic acid and tocopherol, reduce, preferably avoid the formation of N-nitrosamines in the TDS of the present invention by stabilizing the active ingredient in particular during storage.

Plasticizers (softeners) suitable for use in the active ingredient containing layer include fatty acid esters such as decanoyl and octanoyl glycerides or mixtures thereof (such as the product Miglyol^{®} 812), and mixtures of hydrocarbons such as paraffin. A plasticizer is typically contained in the active ingredient containing layer in an amount of 1 to 5 wt.-%, such as e.g. 2.5 wt.-%, based on the overall weight of the (dried) active ingredient containing layer. In an embodiment, the TDS of the present invention does not contain a plasticizer.

Permeation enhancers suitable for use in the present invention include those disclosed in WO'241, i.e. fatty acid esters (e.g. lauryl lactate, isopropyl myristate, oleyl oleate, methyl laurate, isopropyl palmitate, ethyl oleate), fatty alcohols (dodecanol, octyldecanol, lauryl alcohol, alcohols (e.g. propylene glycol), amine oxides (e.g. dimethyldodecyl amine oxide, myristamine oxide), carboxylic acids (e.g. an alpha-hydroxy acid, e.g. lactic acid), caprates (e.g. methyl caprate, propylene glycol dicaprate/dicaprylate), and combinations thereof such as e.g. lauryl lactate/propylene glycol. A permeation enhancer or a combination of permeation enhancers is typically contained in the active ingredient containing layer in an amount of 1 to 5 wt.-%, based on the overall weight of the active ingredient containing layer. However, in the present invention, it is preferred that the active ingredient containing layer does not contain a permeation enhancer. It is particularly preferred that the active ingredient containing layer does not contain the permeation enhancers used in the transdermal therapeutic system disclosed in WO'241.

Cohesion increasing substances suitable for use in the active ingredient containing layer include cationic copolymer from dimethylaminoethylmethacrylate, butylmethacrylate and methyl methacrylate, such as e.g. Eudragit^{®} E100, butyl methacrylate and methylmethacrylate copolymer Plastoid^{®} B, polyvinylpyrrolidone, polyvinylacetate.

Cohesion increasing substances suitable for use in the active ingredient containing layer also include cationic crosslinkers containing cationic aluminium (such as e.g. aluminium actylacetonate) or titanium (such as e.g. titanium butoxide).

Crystallization inhibitors prevent crystallization of the active ingredient in the active ingredient containing layer. Suitable crystallization inhibitors for use in the active ingredient containing layer include polyvinyl pyrrolidone (PVP), polyvinylacetate, and cellulose derivates.

In addition, the active ingredient containing layer may contain a tackifier. Tackifier can preferably be selected from resin from hydrogenated rosin glycerol ester, petroleum-based hydrocarbon resin, polybutene, alicyclic hydrogenated hydrocarbon resin (Arkon^{®} P-100), and mixtures thereof.

If the active ingredient lumateperone is used in salt form, e.g. as tosylate salt, the active ingredient containing layer may comprise further substances that trigger the *in situ* release of lumateperone free base. These substances include inorganic or organic bases, such as alkali metal or alkaline earth metal carbonates, bicarbonates, hydroxides, oxides, and diisopropanolamine.

The absolute amount of the active ingredient in the active ingredient containing layer of the TDS according to the present invention depends on different factors, in particular the size of the TDS to be used, the base weight, and the active ingredient concentration in the active substance containing layer. Exemplary amounts of the active ingredient lumateperone (free base equivalent) to be used in the TDS of the present invention are in the range from 2 to 43.2 mg, e.g. 2 to 28.8 mg, 2 to 27 mg, 2 to 18 mg, 4.5 to 12.5 mg, 6.75 to 18.75 mg, 7.2 to 20 mg, 10.8 to 30 mg, 8 to 11 mg, 12 to 16.5 mg, or 12.8 to 17.6 mg, 29.2 to 26.4 mg. If a salt form of lumateperone is used, these amounts have to be adapted taking into consideration the differing molar weights of lumateperone free base and its salt forms, respectively.

The size of the active ingredient containing layer is typically in the range from 4 to 40 cm². In an embodiment, the size of the active ingredient containing layer is not larger than 36 cm², preferably not larger than 25 cm², more preferably not larger than 22 cm², most preferably not larger than 20 cm², such as e.g. 18, 16, 14, 12, 10, 8, or 6 cm².

The weight of the dried active ingredient containing layer ("matrix weight") is preferably in the range from 20 to 100 g/m², more preferred in the range of 30 to 90 g/m², and still more preferred in the range from 40 to 80 g/m². For a 1 day patch, the weight is typically in the range from 40 to 50 g/m². For a 1 to 2 days patch, the weight is typically in the range from 40 to 60 g/m². For a 2 to 3 days patch, the weight is typically in the range from 50 to 70 g/m². For a 3 to 4 days patch, the weight is typically in the range from 65 to 80 g/m².

Exemplary combinations that take into account the size of the transdermal therapeutic system, the drug loading, and the matrix weight are as follows (with API = active pharmaceutical ingredient = lumateperone free base):
4 cm² TDS, 10% API (2 mg), 50 g/m²
9 cm² TDS, 10% API (4.5 mg), 50 g/m²
16 cm² TDS, 10% API (8 mg), 50 g/m²
20 cm² TDS, 10% API (10 mg), 50 g/m²
22 cm² TDS, 10% API (11 mg), 50 g/m²
25 cm² TDS, 10% API (12.5 mg), 50 g/m²
36 cm² TDS, 10% API (18 mg), 50 g/m²
4 cm² TDS, 15% API (3 mg), 50 g/m²
9 cm² TDS, 15% API (6.75 mg), 50 g/m²
16 cm² TDS, 15% API (12 mg), 50 g/m²
20 cm² TDS, 15% API (15 mg), 50 g/m²
22 cm² TDS, 15% API (16.5 mg), 50 g/m²
25 cm² TDS, 15% API (18.75 mg), 50 g/m²
36 cm² TDS, 15% API (27 mg), 50 g/m²
4 cm² TDS, 10% API (3.2 mg), 80 g/m²
9 cm² TDS, 10% API (7.2 mg), 80 g/m²
16 cm² TDS, 10% API (12.8 mg), 80 g/m²
20 cm² TDS, 10% API (16 mg), 80 g/m²
22 cm² TDS, 10% API (17.6 mg), 80 g/m²
25 cm² TDS, 10% API (20 mg), 80 g/m²
36 cm² TDS, 10% API (28.8 mg), 80 g/m²
4 cm² TDS, 15% API (4.8 mg), 80 g/m²
9 cm² TDS, 15% API (10.8 mg), 80 g/m²
16 cm² TDS, 15% API (19.2 mg), 80 g/m²
20 cm² TDS, 15% API (24 mg), 80 g/m²
22 cm² TDS, 15% API (26.4 mg), 80 g/m²
25 cm² TDS, 15% API (30 mg), 80 g/m²
36 cm² TDS, 15% API (43.2 mg), 80 g/m².

### Adhesive layer

In a preferred embodiment of the present invention, the transdermal therapeutic system of the present invention further comprises an adhesive layer. In this embodiment, the adhesive layer is the skin-contacting layer of the system. The adhesive layer has the effect that it increases the adhesiveness of the system. Furthermore, the adhesive layer increases the duration of the permeation, i.e. it prolongs the time that the transdermal system can be used. The adhesive layer comprises an adhesive polymer and is initially free of the active ingredient lumateperone. "Initially free", as already explained above, means that the adhesive layer is prepared without the active ingredient and that the adhesive layer is accordingly free of the active ingredient before the adhesive layer is laminated with the active ingredient containing layer. When the TDS is manufactured by laminating the active ingredient containing layer with the adhesive layer, a certain amount of the active ingredient is distributing into the initially active ingredient free adhesive layer by diffusion. The adhesive layer is then no longer free of the active ingredient.

The adhesive polymer used in the adhesive layer is preferably selected from the group consisting of acrylate polymers and copolymers, styrene-butadiene-styrene (SBS) copolymers, and polyisobutylene, and mixtures thereof.

In a preferred embodiment, polyacrylate homopolymers, copolymers, and block-copolymers on the basis of acrylic acid esters and/or methacrylic acid esters can be used as adhesive polymers. As monomers for the production of suitable polyacrylates in particular n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethylhexyl methacrylate, isobornyl methacrylate, isobutyl methacrylate, isopropyl acrylate, isopropyl methacrylate and mixtures of these monomers are possible. These monomers are esters of the acrylic or methacrylic acid, respectively, that carry linear, branched or cyclic aliphatic C1-C12 substituents without other free functional groups. Also vinyl acetate can be used as a comonomer together with at least one of these monomers for the production of the polyacrylate.

In a more preferred embodiment, the acrylate polymers and copolymers used as adhesive polymers comprise free hydroxy groups. Examples include the products Duro-Tak^{®} 87-2516 and 387-2516, which are acrylate-vinyl acetate copolymers containing free hydroxy-groups.

In another more preferred embodiment, the acrylate polymers and copolymers used as adhesive polymers comprise free carboxylic acid groups. Examples include the products Duro-Tak^{®} 87-2051 and 387-2051, which are acrylate-vinyl acetate copolymers containing free carboxylic acid groups.

In another preferred embodiment, the adhesive polymer is a styrene-butadiene-styrene copolymer. In a particularly preferred embodiment, the styrene-butadiene-styrene copolymer is the product Duro-Tak^{®} 87-6911, which is a mixture of styrene-butadiene-styrene and styrene-butadiene block copolymers and tackifier(resin) consisting of petroleum-based hydrocarbon resin.

In another preferred embodiment, the adhesive polymer is polyisobutylene. Examples for suitable polyisobutylenes for use as adhesive polymer in the adhesive layer of the TDS of the present invention are the products Duro-Tak^{®} 87-626A and Duro-Tak^{®} 87-6900. Those products are preferably used as a 1:1 mixture.

It has further been found that the adhesive strength of an adhesive layer based on polyisobutylene can be increased by adding polybutene, such as e.g. the products Indopol^{®} H-1900 and H-18000. Indopol^{®} H-1900 is a polybutene with an average molecular weight M*_{N}* of about 2,500 g/mol. Indopol^{®} H-18000 is a polybutene with an average molecular weight M*_{N}* of about 6,000 g/mol. The "average molecular weight M*_{N}*" is the number-average molar mass and can be determined according to American Standard ASTM D3536-91 or ASTM D5296-05. Polyisobutylene and polybutene are preferably used in a weight ratio of 4:1 to 1:2, more preferably 3:1 to 1:2. For example, polyisobutylene and polybutene can be present in a weight ratio of about 1:1.

Preferably, the adhesive layer of the TDS according to the present invention contains 80 to 100 wt.-% of the at least one adhesive polymer, such as e.g. 85 to 100 wt.-%. 90 to 100 wt.-%, 95 to 100 wt.-% or 97.5 to 100 wt.-%, based on the overall weight of the adhesive layer.

The adhesive layer preferably consists of the at least one adhesive polymer. Optionally, it further comprises one or more excipients selected from the group consisting of adhesiveness enhancers, plasticizers, cohesion increasing substances, antioxidants, which are typically contained in an amount of up to 20 wt.%, such as e.g. 15 wt.%, 10 wt.%, 5 wt.%, or 2.5 wt.-%, based on the overall weight of the adhesive layer.

Adhesiveness enhancers increase the adhesive strength of the adhesive layer. Surprisingly, it has been found that the adhesive strength of an adhesive layer based on acrylate polymers and copolymers can be increased by adding Miglyol^{®} 812 in a small amount of e.g. 2 wt.-%, based on the overall weight of the adhesive layer. Miglyol^{®} 812 is typically used as a plasticizer (softener), and not as an adhesiveness enhancer.

The weight of the (dried) adhesive layer is typically in the range from 10 to 50 g/m², preferably in the range from 20 to 40 g/m², such as e.g. 30 g/m².

### Membrane layer

In a third aspect of the present invention, a membrane layer between the active ingredient containing layer and the adhesive layer is used, wherein the membrane layer comprises a membrane polymer that is permeable for the active ingredient and controls the release of the active ingredient. The membrane layer enables the prolongation of the constant permeation time for several days. At the same time, however, permeation is reduced compared to a TDS without membrane layer.

Preferably, the membrane polymer consists of a polyolefine such as for example polypropylene (PP) (e.g., Celgard^{®} 2400), or of polyethylene (PE) (e.g., CoTran^{™} 9719 or CoTran^{™} 9720), or of polyethylene with a vinyl acetate proportion (EVA), such as a vinyl acetate proportion of e.g. 4.5 to 19% (e.g. CoTran^{™} 9707; CoTran^{™} 9702; CoTran^{™} 9728). Moreover, the membranes can have a porosity of up to 90% (e.g. Solupor^{®} 10P05A, Celgard^{®} 2400).

Porous membranes or coherent membranes may be used.

The porosity of the porous membranes can be up to about 90%. Examples of preferred porous membranes are:
Solupor^{®} 10P05A (polyethylene, porosity: 83%, thickness: 60 µm)
Celgard^{®} 2400 (polypropylene, porosity: 41%, thickness: 25 µm)

Typically, the membrane has a thickness of 0.01 and 0.15 mm. The preferred thickness of the membrane is 0.020 to 0.080 mm.

According to the invention, it is particularly preferred to use a coherent membrane substantially consisting of polyethylene with a thickness of about 40 to 50 µm (e.g. CoTran^{™} 9719), which enables prolongation of the constant permeation time for up to 3 days; or a porous membrane consisting of polypropylene with a thickness of about 20 to 40 µm (e.g. Celgard^{®} 2400), which enables prolongation of the constant permeation time for up to three days, but on the other hand maintaining a sufficiently high permeation to allow a small patch size of e.g. about 36 or, preferably, 20 cm² or smaller.

### Backing layer

The TDS of the present invention comprises a backing layer. Typically, the backing layer of the TDS according to the invention is occlusive, that is it is the outermost layer in the cross-section of the finished TDS which is furthest away from the skin contacting layer. The backing layer is an inert layer which is substantially free of the active ingredient and substantially impermeable for the active ingredient.

In a preferred embodiment, such backing layers consist of polyolefins, in particular polyethylene, or polyesters as well as polyurethanes. Also layers containing several different polymers arranged on top of each other can preferably be used. Suitable materials comprise polyolefin, cellophane, cellulose acetate, ethyl cellulose, vinyl acetate-vinyl chloride copolymers provided with plasticizers, ethylene-vinylacetate copolymers, polyethylene terephthalate, nylon, polyethylene, polypropylene, polyvinylidene chloride, ethylene-methacrylate copolymers, paper which can optionally be coated, textile tissue, aluminum foil, and polymer-metal composite materials. Polyester foils, such as polyethylene terephthalate foils, e.g. the product Hostaphan^{™} MN 19 are particularly preferred.

Especially preferred are composite foils of tan pigmented polyethylene, thermoplastic resin and aluminum vapor coated polyester, e.g. the product Scotchpak^{™} 9738 or lacquered metalized Hueck PET foils. Polymer-metal composite materials, such as e.g. the product Scotchpak^{™} 9738, protect the active ingredient against light-induced degradation in particular during storage. Polymer-non-metal composite materials, e.g. Scotchpak^{™} 9723 or lacquered Hueck PET foils, can also be used in case the composition of the backing layer sufficiently inhibits light-induced degradation through e.g. pigments or excipients within a lacquer or outer layer.

As is common in the prior art, the thickness of the back layer may be for example 10 µm to 100 µm, for example about 40 µm (nominal thickness). This ensures flexibility and wearing comfort of the transdermal therapeutic system of the present invention.

### Release liner

In a preferred embodiment of the present invention, the skin-contacting layer of the TDS, i.e. the active ingredient containing layer (first aspect) or the adhesive layer (second and third aspect) is protected with a release liner. The release liner is deposited on the skin-contacting layer and is removed when the TDS is to be used.

Preferably, the release liner is prepared from polymeric material that optionally can also be metallized. Examples of preferably used materials are polyurethanes, polyvinylacetate, polyvinylidene chloride, polypropylene, polycarbonate, polystyrene, polyethylene, polyethylene terephthalate, polybutylene terephthalate as well as paper that is optionally surface coated with corresponding polymers. The release liner can be coated, e.g. fluoropolymer or non-fluoropolymer-coated, silicone- or fluorosilicone-coated on one or both sides, wherein siliconized release liners are particularly suitable for polyacrylate-based layers. Particularly preferred are coated polyester foils, such as the one-sided siliconized commercial products Primeliner^{™} 75 or 100 µm and Perlasic LF 75 µm (Loparex, NL and Perlen Converting AG, Switzerland) and the silicon-coated polyethylenetherepthalate (PET) (e.g. Loparex Primeliner^{™} 78HL), or the one-sided fluoropolymer-coated products such as e.g. Scotchpak^{™} 1022 (3M Drug delivery). Further suitable products are Scotchpak^{™} 9744 and Scotchpak^{™} 9709.

### Method for the preparation of the TDS according to the present invention

A further aspect of the present invention is to provide a method for the production of the TDS according to the invention. The method comprises the following steps:
(a) preparing an active ingredient containing layer composition,
(b) coating the composition prepared in step (a) onto a release liner (i),
(c) drying the component prepared in step (b), and
(d) laminating a backing layer onto the active ingredient layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa*;
or optionally comprising the following steps in addition to steps (a)-(d):
(j) laminating a membrane layer suitable to control the release of the active ingredient onto the active ingredient containing layer of the component prepared in accordance with step (h),
(k) performing steps (e) to (g),
(I) laminating the adhesive layer of the component prepared in step (k) onto the membrane layer of the component prepared in step (j), or *vice versa.*

In general, the expressions "active ingredient containing layer composition", "adhesive layer composition", and "membrane layer composition" mean that they comprise the same ingredients as described above for the "active ingredient containing layer", the "adhesive layer", and the "membrane layer". In addition thereto, they may comprise a suitable organic solvent, such as e.g. heptane or ethyl acetate, but other organic solvent or solvent mixtures known to the skilled person are also possible. Preferably, a volatile organic solvent or solvent mixture is used in the preparation of the respective compositions.

The preparation of the TDS of the present invention may be performed by firstly dispersing or dissolving, respectively (unless the polymer is already dissolved) the components for the active ingredient containing layer, that is the active ingredient and the matrix-forming polymer or copolymer, respectively, or a mixture thereof, in a suitable organic solvent as described above, such as e.g. heptane or ethyl acetate, or other organic solvent or solvent mixtures known to the skilled person. Preferably, a volatile organic solvent or solvent mixture is used. Optionally, further excipients as described in connection with the active ingredient containing layer are added. Typically, the matrix-forming polymer or copolymer or the mixture thereof is already present in a solvent. Here, according to the invention a polymer and/or copolymer is used that is as defined above in connection with the TDS according to the invention. The embodiments of the polymer matrix mentioned above as being preferred correspondingly apply to the method according to the invention.

Then, said mixture is applied onto a release liner (i) as a uniform layer and dried.

In the next step, a backing layer is applied to the active ingredient containing layer.

In a separate step, the adhesive layer is prepared by dispersing the polymer mixture forming the contact adhesive (and dissolved in an organic solvent), optionally together with further excipients as described in connection with the adhesive layer, in a suitable organic solvent such as e.g. heptane or ethyl acetate, but also other organic solvent or solvent mixtures known to the skilled person are possible. Preferably, a volatile organic solvent or solvent mixture is used. Then, said mixture is applied onto a release liner (ii) and allowed to dry. The embodiments of the adhesive layer mentioned above as being preferred correspondingly apply to the method according to the invention.

The components obtained in these two process steps are subsequently laminated together, namely such that the adhesive layer is directly applied to the active ingredient containing layer after the release liner (i) has been removed. In the embodiments where a membrane layer controlling the release of the active ingredient is used, the adhesive layer is laminated to the membrane, or *vice versa.*

In case that a membrane controlling the release of the active substance is to be applied, after the active ingredient containing layer is dried this can be applied to the side of the active ingredient containing layer after the release liner (i) has been removed. The adhesive layer is then laminated onto the membrane layer, or *vice versa.* Subsequently, pieces of the desired size can be punched from the finished laminate and packaged.

In the individual process steps, the organic solvents required to dissolve or disperse the respective components are removed by subjecting the products to a drying step by use of increasing temperatures, optionally also using a partial vacuum.

The preparation of the transdermal therapeutic systems in accordance with the present invention is further illustrated in the examples.

### Further aspects and embodiments

Preferably, no permeation enhancer is used in the TDS of the present invention. It is particularly preferred that the TDS of the present invention does not contain the permeation enhancers used in the transdermal therapeutic system disclosed in WO'241.

The size of the TDS of the present invention is not particularly limited, but is typically in the range from 4 to 40 cm². In an embodiment, the size of the TDS of the present invention is not larger than 36 cm², preferably not larger than 25 cm², more preferably not larger than 22 cm², most preferable not larger than 20 cm², such as e.g. 18, 16, 14, 12, 10, 8, 6 cm².

The daily amount of the active ingredient lumateperone delivered transdermally *in vivo* is preferably at least about 1.5 mg/day, preferably about 1.8 mg/day. This corresponds to a daily oral dose of 60 mg lumateperone tosylate being equivalent to 42 mg lumateperone free base, which is required taking into account the low oral bioavailability of about 4.4% of Caplyta^{®}.

In accordance therewith, the flux is preferably at least 3.75 µg/cm²*h. For a patch having a preferred size of 20 cm², this results in a daily amount of at least 1.5 mg/day. This flux is preferably achieved without the use of permeation enhancers in the TDS. The application period of a TDS according to the invention is at least one day, preferably at least 2, more preferably at least 3 days. The TDS according to the first aspect of the present invention is preferably suitable for an application period of 2 to 3 days, more preferably 3 to 4 days. The TDS according to the second aspect of the present invention is preferably suitable for an application period of 2 to 3 days, more preferably 3 to 4 days. The TDS according to the third aspect of the present invention is preferably suitable for an application period of 2 to 3 days, more preferably 3 to 4 days.

Preferably, the TDS according to the invention has a constant permeation profile for several days. Here, the permeation is constant over a period of at least 24 hours (1 day), preferably at least 48 hours (2 days), more preferably at least 72 hours (3 days).

The TDS according to the first aspect of the present invention preferably has a constant permeation profile over a period of preferably 2 days, more preferably 3 days.

In a further aspect, the present invention concerns the use of (1) a polyacrylate, preferably a polyacrylate containing free hydroxy groups, (2) a mixture of a polyacrylate and a silicone polymer, and (3) a silicone polymer for stabilizing lumateperone free base in a transdermal therapeutic system, or reducing, preferably preventing degradation of lumateperone free base in a transdermal therapeutic system, preferably over a time period of at least 6 months, more preferably over a time period of at least 12 months at room temperature. The transdermal therapeutic system is preferably the TDS according to the present invention in terms of all of its aspects and embodiments. The use of a polyacrylate containing free hydroxy groups is preferable. The preferred polyacrylate containing free hydroxy groups is an acrylate vinyl-acetate copolymer such as Duro-Tak^{®} 387-2516.

In a further aspect of the present invention, the transdermal therapeutic system of the present invention is for use in a method for treating a disease selected from the group consisting of major depressive disorder (MDD) and adjunctive mixed features associated therewith, bipolar depression I or II and schizophrenia.

In a further aspect of the present invention, lumateperone is for use in a method for treating a disease selected from the group consisting of major depressive disorder (MDD) and adjunctive mixed features associated therewith, bipolar depression I or II and schizophrenia, wherein lumateperone is administered in free base form or salt form through the skin by means of the transdermal therapeutic system of the present invention.

### Specific embodiments

In a specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a polyacrylate with free hydroxy groups, preferably an acrylate-vinyl acetate copolymer containing free hydroxy groups (such as e.g. the product Duro-Tak^{®} 387-2516); the active ingredient containing layer preferably contains 0.05-0.2 wt.-% of the antioxidant tocopherol, ascorbyl palmitate or a combination thereof in order to increase the chemical stability of the active ingredient; the active ingredient containing layer optionally contains a mixture of decanoyl and octanoyl glycerides (such as e.g. Miglyol^{®} 812) as a plasticizer in an amount of 1-5 wt.-%, such as e.g. 2.5 wt-%, in order to increase the adhesiveness of the system; a permeation enhancer as mentioned herein can be added, however, it is preferable that no permeation enhancer is added; for obtaining a 3 to 4 days patch, the matrix weight is preferably 65-80 g/m²;
c. a release liner, which is preferably a siliconized polyester foil.

In a further specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a silicone polymer, preferably a mixture of a high tack silicone polymer (such as e.g. the product BIO-PSA 7-4302) and a medium tack silicone polymer (such as e.g. the product BIO-PSA 7-4202) in a ratio of 70-60:30-40; or, alternatively a mixture of the high tack silicone polymer BIO-PSA SRS7-4602 and the medium tack silicone polymer BIO-PSA SRS7-4502 in a ratio of 70-60:30-40; the active ingredient containing layer preferably contains 0.05-0.2 wt.-% of the antioxidant tocopherol, ascorbyl palmitate or a combination thereof in order to increase the chemical stability of the active ingredient; the active ingredient containing layer can contain paraffine as a plasticizer in an amount of 1-3 wt.-% in order to increase the adhesiveness of the system; for obtaining a 2 to 3 days patch, the matrix weight is preferably 60-70 g/m²;
c. a release liner, which is preferably a fluorosiliconized or a non-fluoro containing polyester foil.

In a further specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a mixture of an acrylate polymer (such as e.g. the product Gelva GMS 788) and a silicone polymer (e.g. the product BIO-PSA 7-4202) in a ratio of 1:1.5 to 1:3, preferably 1:2; the active ingredient containing layer preferably contains 0.05-0.2 wt.-% of the antioxidant tocopherol, ascorbyl palmitate, or a combination thereof in order to increase the chemical stability of the active ingredient; or obtaining a 2 to 3 days patch, the matrix weight is preferably 65-80 g/m²;
c. a release liner, which is preferably a fluoropolymer-coated or fluorosiliconized, or a non-perfluoro or non-polyfluoro polyester foil.

In a further specific embodiment, the TDS of the present invention comprises (in accordance with the second aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a polyacrylate with free hydroxy groups, preferably an acrylate-vinyl acetate copolymer containing free hydroxy groups (such as e.g. the product Duro-Tak^{®} 387-2516); the active ingredient containing layer preferably contains 0.05-0.2 wt.-% of the antioxidant tocopherol, ascorbyl palmitate or a combination thereof in order to increase the chemical stability of the active ingredient; the active ingredient containing layer optionally contains a mixture of decanoyl and octanoyl glycerides (such as e.g. Miglyol^{®} 812) as a plasticizer in an amount of 1-5 wt.-%, such as e.g. 2.5 wt-%, in order to increase the adhesiveness of the system; a permeation enhancer as mentioned herein can be added, however, it is preferably that no permeation enhancer is added; for obtaining a 3 to 4 days patch, the matrix weight is preferably 65-80 g/m²;
c. an adhesive layer, comprising a polyacrylate adhesive with free hydroxy groups (such as e.g. Duro-Tak^{®} 387-2516), preferably with a small amount of e.g. 2 wt.-% a of a mixture of decanoyl and octanoyl glycerides (such as e.g. Miglyol^{®} 812) added in order to increase the adhesiveness of the layer; alternatively comprising a styrene-butadiene-styrene (SBS) copolymer adhesive (such as e.g. the product Duro-Tak^{®} 87-6911); or alternatively containing a mixture of one or more polyisobutylene (such as e.g. the products Duro-Tak^{®} 87-626A and Duro-Tak^{®} 87-6900 in a ratio of 1:1 or 3:2) and one or more polybutenes (such as e.g. the products Indopol H-1900 and Indopol H-18000 in a ratio of 1:1 or 3:2), wherein the polyisobutylene/polybutene ratio is 1:1); for obtaining a 3 to 4 days patch, the weight of the adhesive layer is preferably about 30 g/m².
d. a release liner, which is preferably a siliconized polyester foil.

In a further specific embodiment, the TDS of the present invention comprises (in accordance with the third aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a polyacrylate with free hydroxy groups, preferably an acrylate-vinyl acetate copolymer containing free hydroxy groups (such as e.g. the product Duro-Tak^{®} 387-2516); the active ingredient containing layer preferably contains 0.05-0.2 wt.-% of the antioxidant tocopherol, ascorbyl palmitate or a combination thereof in order to increase the chemical stability of the active ingredient; the active ingredient containing layer optionally contains a mixture of decanoyl and octanoyl glycerides (such as e.g. Miglyol^{®} 812) as a plasticizer in an amount of 1-5 wt.-%, such as e.g. 2.5 wt-%, in order to increase the adhesiveness of the system; a permeation enhancer as mentioned herein can be added, however, it is preferably that no permeation enhancer is added; for obtaining a 3 to 4 days patch, the matrix weight is preferably about 65 to 80 g/m²;
c. a membrane layer consisting of coherent polyethylene (such as e.g. the product Cotran^{™} 9719), or alternatively, consisting of porous polypropylene (such as e.g. the product Celgard^{™} 2400).
d. an adhesive layer, comprising a polyacrylate adhesive with free hydroxy groups (such as e.g. Duro-Tak^{®} 387-2516), preferably with a small amount of e.g. 2 wt.-% a mixture of decanoyl and octanoyl glycerides (such as e.g. Miglyol^{®} 812) added in order to increase the adhesiveness of the layer; alternatively comprising a styrene-butadiene-styrene (SBS) copolymer adhesive (such as e.g. the product Duro-Tak^{®} 87-6911); or alternatively containing a mixture of one or more polyisobutylenes (such as e.g. the products Duro-Tak^{®} 87-626A and Duro-Tak^{®} 87-6900 in a ratio of 1:1 or 3:2) and one or more polybutenes (such as e.g. the products Indopol H-1900 and Indopol H-18000 in a ratio of 1:1 or 3:2), wherein the polyisobutylene/polybutene ratio is 1:1); for obtaining a 3 to 4 days patch, the weight of the adhesive layer is preferably about 30 g/m².
e. a release liner, which is preferably a siliconized polyester foil.

### Examples

### Example 1: Monolayer formulation based on a polyacrylate adhesive (first aspect of the invention)

### a) Preparation

### Samples 1-8

An active ingredient containing layer containing the active ingredient lumateperone free base, a polyacrylate adhesive comprising free hydroxy groups (Duro-Tak^{®} 387-2516), and, optionally, further excipients (Miglyol^{®} 812), was coated on a protective film (Primeliner^{™} 78HL). The solvents were removed in a drying oven. The active layer was then laminated with a backing layer (Hostaphan^{™} MN 19 Med or Scotchpak^{™} 9738).

### Samples 9-14

An active ingredient containing layer containing the active ingredient lumateperone free base, a polyacrylate adhesive comprising either no functional groups (Duro-Tak^{®} 87-4098) or free carboxylic acid groups (Duro-Tak^{®} 387-2054), and, optionally, further excipients (oleyloleat and dodecanol), was coated on a protective film (Primeliner^{™} 78HL). The solvents were removed in a drying oven. The active layer was then laminated with a backing layer (Hostaphan^{™} MN 19 Med or Scotchpak^{™} 9738).

The resulting laminates are shown in the following **Table 1.**

**Table 1**

| **No.** | **Batch** | **Composition of the (dried) active ingredient containing (matrix) layer** | **Matrix weight [g/m²]** | **Backing layer/ Release liner** |
|---|---|---|---|---|
| 1 | 001LUMTDS | 10% Lumateperone | 50 | Hostaphan MN12/ Primeliner 78HL |
| | | 90% Duro-Tak^{®} 387-2516 | | |
| 2 | 062LUMTDS | 10% Lumateperone | 65 | Scotchpak 9738/ Primeliner 78HL |
| | 062LUMTDS | 90% Duro-Tak^{®} 387-2516 | 80 | |
| 3 | 014LUMTDS | 15% Lumateperone | 50 | Scotchpak 9738/ Primeliner 78HL |
| | 033LUMTDS | 85% Duro-Tak^{®} 387-2516 | 50 | |
| 4 | 061LUMTDS | 15% Lumateperone | 65 | Scotchpak 9738/ Primeliner 78HL |
| | 061LUMTDS | 85% Duro-Tak^{®} 387-2516 | 80 | |
| 5 | 022LUMTDS | 15% lumateperone | 50 | Scotchpak 9738/ Primeliner 78HL |
| | | 2.5% Miglyol^{®} 812 | | |
| | | 82.5% Duro-Tak^{®} 387-2516 | | |
| 6 | 023LUMTDS | 15% Lumateperone | 50 | Scotchpak 9738/ Primeliner 78HL |
| | | 15% Eudragit E100 | | |
| | | 70% Duro-Tak^{®} 387-2516 | | |
| 7 | 024LUMTDS | 15% Lumateperone | 50 | Scotchpak 9738/ Primeliner 78HL |
| | | 15% Duro-Tak^{®} 387-2051 | | |
| | | 70% Duro-Tak^{®} 387-2516 | | |
| 8 | 086LUMTDS | 15% Lumateperone | 50 | Scotchpak 9738/ Primeliner 78HL |
| | | 15% Plastoid B | | |
| | | 70% Duro-Tak^{®} 387-2516 | | |
| | | | | |
| 9 | 002LUMTDS | 10% Lumateperone | 50 | Hostaphan MN12/ Primeliner 78HL |
| | | 90% Duro-Tak^{®} 87-4098 | | |
| 10 | 007LUMTDS | 10% Lumateperone | 50 | Hostaphan MN12/ Primeliner 78HL |
| | | 10% Dodecanol | | |
| | | 80% Duro-Tak^{®} 87-4098 | | |
| 11 | 008LUMTDS | 10% Lumateperone | 50 | Hostaphan MN12/ Primeliner 78HL |
| | | 10% Oleyloleat | | |
| | | 80% Duro-Tak^{®} 87-4098 | | |
| 12 | 015LUMTDS | 15% Lumateperone | 50 | Hostaphan MN12/ Primeliner 78HL |
| | | 85% Duro-Tak^{®} 87-4098 | | |
| 13 | 003LUMTDS | 10% Lumateperone | 50 | Hostaphan MN12/ Primeliner 78HL |
| | | 90% Duro-Tak^{®} 387-2054 | | |
| 14 | 025LUMTDS | 15% Lumateperone | 50 | Scotchpak 9738/ Primeliner 78HL |
| | | 85% Duro-Tak^{®} 387-2054 | | |

### b) Crystallization and phase system

Some of the samples prepared in Example 1 were analyzed for the occurrence of crystallization and the presence of a biphasic system after being stored at 40°C/75% RH. The results are shown in the following **Table 2.**

**Table 2**

| **No.** | **Batch** | **Storage conditions** | **crystallization visible?** | **Biphasic system visible (using a microscope)?** |
|---|---|---|---|---|
| 1 | 014LUMTDS | 6 months at 40°C/75% RH | no | no |
| 2 | 024LUMTDS | 6 months at 40°C/75% RH | no | no |

| | | | | |
|---|---|---|---|---|
| rt = room temperature; RH = relative humidity | | | | |

### c) Adhesive strength

The adhesion strength can be determined as the force required to detach a specimen from a suitable support at a specified angle and at a defined speed. For the determination of the adhesion strength, TDS of a defined size, e.g. 10 cm², are punched out and conditioned at 23 ± 1°C and 50 ± 5% RH. A conductive strip, e.g. 10 cm², is applied to the TDS. The conductive strip consists of, e.g. double-sided adhesive tape. The TDS is applied to a test plate, e.g. made of steel, with its release liner removed and then pressed between two glass plates for e.g. 1 min with a 2 kg weight. The test plate is fixed horizontally in a tensile testing machine, e.g. Texture Analyzer plus from Stable Micro Systems, and the liner is clamped so that the TDS is pulled off at a 90° angle. The measurement is made at a specified speed of usually 300 ± 30 mm/min at typically 23 ± 1°C and 50 ± 5% RH. The mean force [N/25 mm], normalized to a specimen width of 25 mm, measured over the distance is the adhesive force.

The adhesive strength was determined after 0 months, 1 months, 3 months and 6 months at 40°C/75% RH for some of the transdermal therapeutic systems prepared in Example 1.

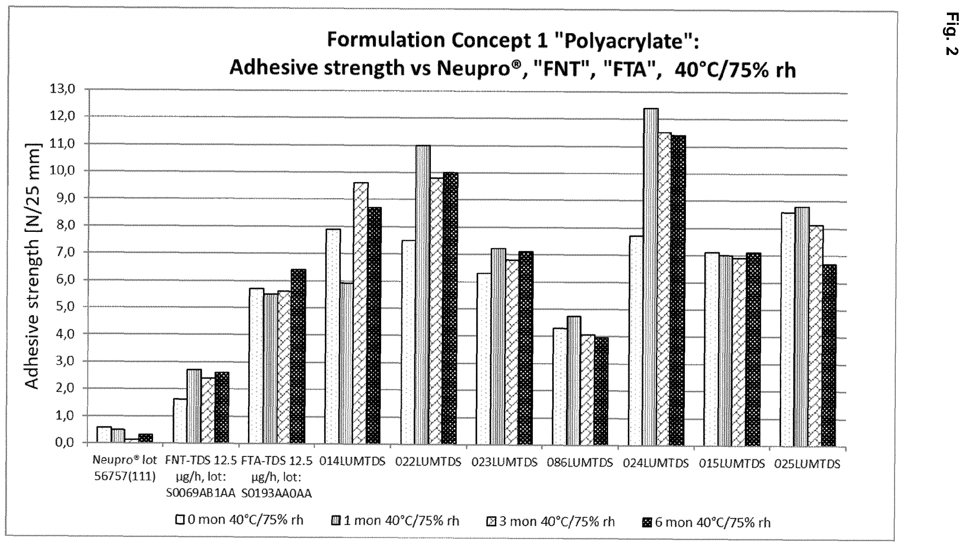

The results are shown in **Fig. 2****.**

From **Fig. 2****,** it can be taken that the TDS comprising lumateperone free base in combination with a polyacrylate adhesive with free hydroxy groups (Duro-Tak^{®} 387-2516) has a higher adhesive strength than the 1-day patch Neupro^{®} and the 3-day patches FNT and FTA (cf. batch no. 014LUMTDS vs. Neupro^{®} lot, FNT-TDS and FTA-TDS).

Especially after 3 months and 6 months storage at 40°C/75% RH, the TDS comprising lumateperone free base in combination with the polyacrylate adhesive with free hydroxy groups (Duro-Tak^{®} 387-2516) has a higher adhesive strength compared to the respective TDS comprising a polyacrylate adhesive with no functional groups (cf. batch no. 014LUMTDS vs. 015LUMTDS) and a polyacrylate adhesive with free carboxylic groups including a crosslinker (Duro-Tak^{®} 387-2054), respectively (cf. batch no. 014LUMTDS vs. 025LUMTDS).

Surprisingly, a further increase in adhesive strength can be achieved by adding the plasticizer Miglyol^{®} 812 at a low concentration of about 1.5-3% (cf. batch no.'s 014LUMTDS vs. 022LUMTDS).

The addition of a polyacrylate adhesive with free carboxyl acid groups (Duro-Tak^{®} 387-2051) in a concentration of about 15 % also increases the adhesive strength (cf. batch no. 014LUMTDS vs. 024LUMTDS).

The addition of about 15% Eudragit E100 leads to a slight reduction in adhesive strength (cf. batch no.'s 014LUMTDS vs. 023LUMTDS). The addition of about 15% Plastoid^{®} B leads to a significant reduction in adhesive strength (cf. batch no. 014LUMTDS vs. 086LUMTDS).

### d) Tack

Tack can be determined as the maximum force required to completely separate a stainless steel specimen from the adhesive layer of a TDS. For the determination of tack, a patch or laminate is conditioned at 23 ± 1°C and 50 ± 5% RH and subsequently fixed to a perforated carrier plate with its release liner removed and the adhesive matrix open. The plate is fixed in a tensile testing machine, e.g. Texture Analyser plus from Stable Micro Systems. At usually 23 ± 1°C and 50 ± 5% RH, the test specimen is pressed onto the top of the specimen and peeled off after a defined contact time of usually 2 sec. A series of measurements should be completed within 30 min after removal of the release liner from the first specimen. The maximum force (Tack; [N]) to separate the bond between the specimen and the adhesive layer is determined.

Tack was determined after 0 months, 1 months, 3 months and 6 months at 40°C/75% RH for some of the transdermal therapeutic systems prepared in Example 1.

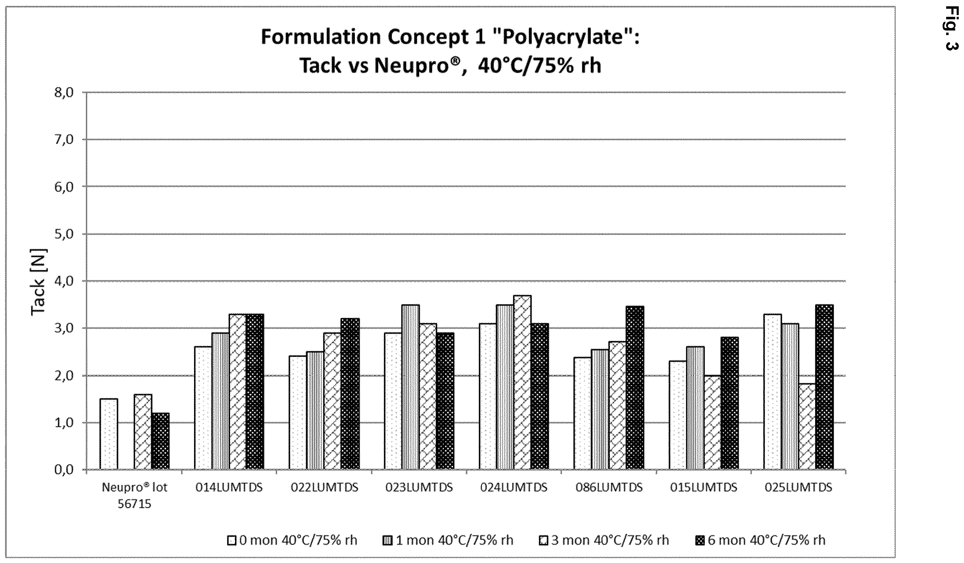

The results are shown in **Fig. 3****.**

From **Fig. 3****,** it can be taken that lumateperone free base in combination with a polyacrylate adhesive with free hydroxy groups (Duro-Tak^{®} 387-2516) (014LUMTDS), with addition of 2.5 % Miglyol^{®} 812 (022LUMTDS), or 15 % Eudragit E100 (023LUMTDS), or 15 % Plastoid B (086LUMTDS), or a non-crosslinked polyacrylate adhesive with free carboxyl acid groups without a crosslinker (Duro-Tak^{®} 387-2051) (024LUMTDS), or a crosslinked polyacrylate adhesive with free carboxylic acid groups (DuroTak^{®} 387-2054) (025LUMTDS), or a polyacrylate adhesive without functional groups (Duro-Tak^{®} 87-4098) (015LUMTDS) show a significantly higher tack than the 1-day patch Neupro.

There is no relevant decrease in tack during storage over 6 months at 40°C/75% RH.

### e) Separation force

The separation force can be determined as the force required to detach a sample from its release liner at a specified angle and speed. For its determination, TDSs' of a defined size, e.g. 10 cm², are punched out and conditioned at 23 ± 1°C and 50 ± 5% RH. A conductive strip, in the form of a liner, is cut out. A guide strip, the width of the TDS, is attached to the TDS. Then the TDS is fixed with the release liner facing downwards on an apparatus slide using double-sided adhesive tape. This is inserted into a tensile testing machine, e.g. Texture Analyser plus from Stable Micro Systems, in such a way that the TDS is pulled off at an angle of 90°. The speed is typically 300 ± 30 mm/min and is usually performed at 23 ± 1°C and 50 ± 5% RH. The average force [N/25 mm], normalized to a specimen width of 25 mm, measured across the separation distance is the separation force.

The separation force was determined after 0 months, 1 months, 3 months and 6 months at 40°C/75% RH for some of the transdermal therapeutic systems prepared in Example 1.

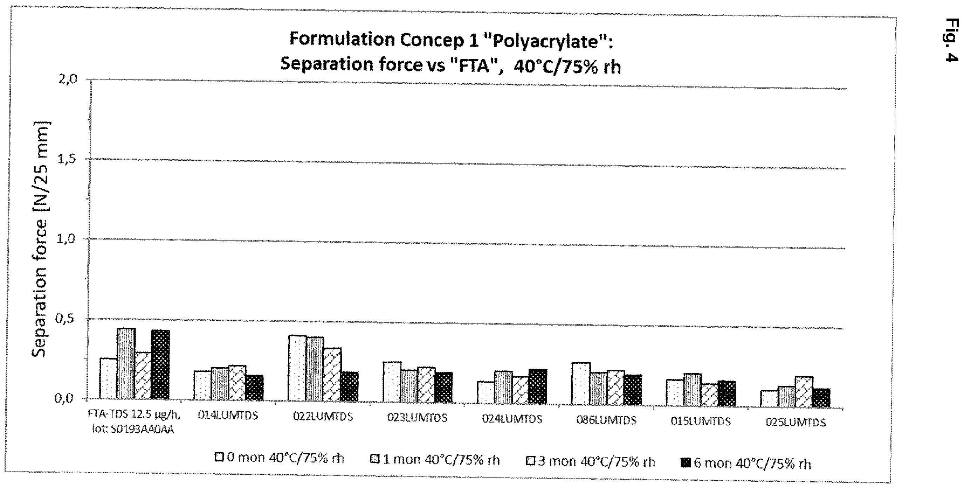

The results are shown in **Fig. 4****.**

From **Fig. 4****,** it can be taken that lumateperone free base in combination with polyacrylate adhesives with free hydroxy groups (014LUMTDS), as well as with further addition of 2.5 % Miglyol^{®} 812 (022LUMTDS), or 15 % Eudragit E100 (023LUMTDS), or 15 % Plastoid B (023LUMTDS), or 15 % of a polyacrylate adhesive with free carboxylic acid groups (Duro-Tak^{®} 387-2051) (024LUMTDS) leads to low separation forces, which moreover do not show any relevant increase in separation force during storage over 6 months at 40°C/75% RH by use of a siliconized polyester release liner.

### f) Permeation

### aa)Preliminary remarks

In order to realize a small patch size, the cumulative permeated amount per 24 h must be sufficiently high. An amount of about 1.8 mg lumateperone must be provided transdermally at an oral bioavailability of about 4.4% per day. For a patch size of about 20 cm², a flux of at least 3.75 µg/cm²*h must be achieved (3.75 µg/cm²*h x 20 cm² x 24 = 1,800 µg/24 h = 1.8 mg/24 h), which corresponds to a transdermally delivered amount of 90 µg per day and cm². Ideally, this flux is achieved without the use of permeation enhancers.

### bb)Permeation on human skin model (HSE)

The permeation of selected samples prepared in Example 1 was measured on human skin model in order to determine the cumulatively permeated amount of lumateperone per cm² for a time period of up to 96 hours.

The *in vitro* skin permeation studies were performed using a skin permeation system from NovoCell Schönbach according to OECD (2004) Test Guideline 428 "Skin absorption: In vitro Method & Series on testing and assessment", No. 28 "Guidance document for the conduct of skin absorption studies".

A measuring cell was kept at 32 ± 1 °C throughout the measurement. The measuring cell consists of a donor and acceptor chamber, which are separated from each other by a heat-separated epidermis of human skin with an effective permeation area of 1.05 cm² lying on a cellulose membrane. The matrix of the patch to be tested (size about 1.2 cm²) was adhered to the stratum corneum, with the surface to be released facing the acceptor chamber. The measuring cell contained a total volume of 15 mL and was filled with phosphate buffer pH 4.5. At defined time points (e.g., 1, 2, 3, 6, 9, 12, 24, 36, 48, 72 and 96 hours), aliquots were taken as samples from the acceptor chamber, RP-HPLC analysis was used to determine the concentration of lumateperone, and immediately removed aliquot was replaced with fresh buffer. A homogeneous distribution of temperature and concentration of lumateperone was ensured by an integrated magnetic stirring system in the acceptor chamber.

RP-HPLC analysis and calculation of sample concentrations were performed. Stationary phase: C18 (e.g. 50x3 mm, 5 µm particle size, 30°C oven temperature). Mobile phase: 10mM phosphate buffer (pH 2.5)/methanol; 55/45 (v/v), with flow 0.7 mL/min. Injection volume: 10 µL, with detection at 229 nm, retention time lumateperone about 3-4 minutes, run time 5 minutes (isocratic). Evaluation was performed via 1-point calibration using external standard solution. Calculation of cumulative release [%] based on determined concentration in sample solutions.

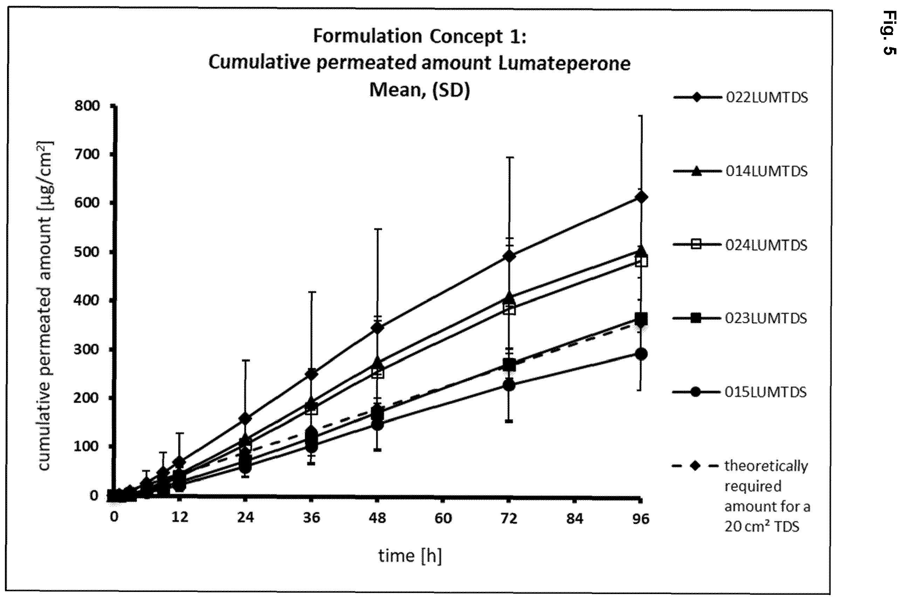

Subsequently, the cumulative permeated amount per time point was calculated and plotted against time in a graph. Then the steady state flux can be calculated [µg/cm²/h]. The results are depicted in **Fig. 5****.**

The samples show the following permeation effect on human skin model (HSE) from high to low in **Fig. 5****:**
2.5% Miglyol^{®} 812, 82.5% Duro-Tak^{®} 387-2516 (022LUMTDS) >
85% Duro-Tak^{®} 387-2516 (014LUMTDS) ~
70% Duro-Tak^{®} 387-2516, 15% Duro-Tak^{®} 387-2051 (024LUMTDS) >
15% Eudragit E100, 70% Duro-Tak^{®} 387-2516 (023LUMTDS) >
85% Duro-Tak^{®} 87-4098 (015LUMTDS).

The dashed line in **Fig. 5** indicates the theoretically required amount of lumateperone for a 20 cm² matrix. All of the samples comprising a polyacrylate adhesive with free hydroxy groups (022LUMTDS, 014LUMTDS, 024LUMTDS, 023LUMTDS) are in accordance with this requirement.

The permeation for these patches (50 mg/cm² patches at 15% drug load) is constant for 2 to 3 days.

### cc) Optimization of drug concentration and matrix weight

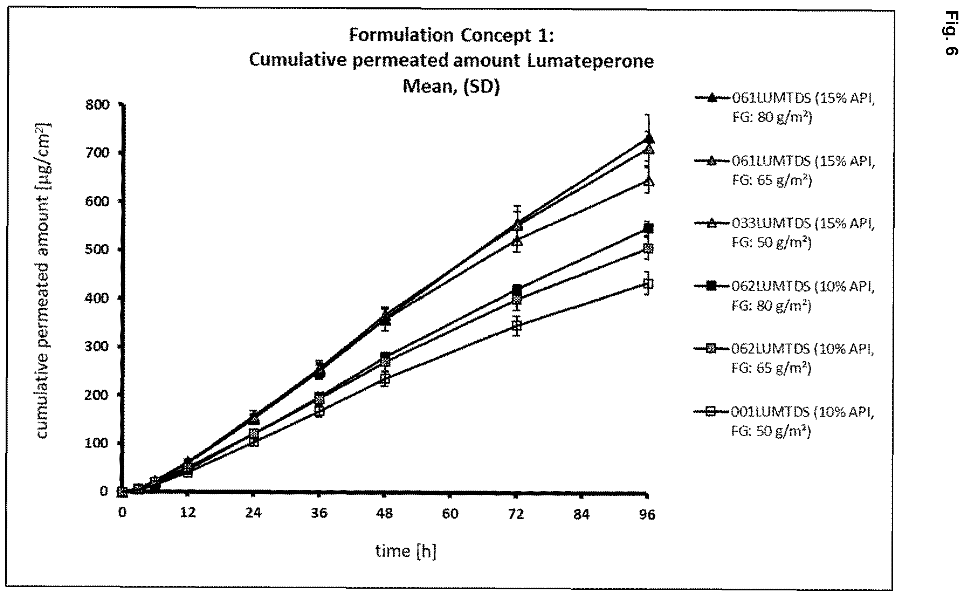

For the purpose of matrix weight optimization, selected samples with different drug concentrations and different matrix weights prepared in Example 1 were compared in terms of their permeation on human skin model (HSE). The results are depicted in **Fig. 6****.**

The samples show the following permeation effect on human skin model (HSE) from high to low in **Fig. 6****:**
15% Lumateperone, 85% Duro-Tak^{®} 387-2516, 80 g/m² (061LUMTDS) >
15% Lumateperone, 85% Duro-Tak^{®} 387-2516, 65 g/m² (061LUMTDS) >
15% Lumateperone, 85% Duro-Tak^{®} 387-2516, 50 g/m² (033LUMTDS) >
10% Lumateperone, 90% Duro-Tak^{®} 387-2516, 80 g/m² (062LUMTDS) >
10% Lumateperone, 90% Duro-Tak^{®} 387-2516, 65 g/m² (062LUMTDS) >
10% Lumateperone, 90% Duro-Tak^{®} 387-2516, 50 g/m² (001LUMTDS)

### g) Chemical Stability

The samples according to the following **Table 3** were prepared in accordance with the procedures described under a):

**Table 3**

| **No.** | **Batch** | **Composition of the (dried) active ingredient containing (matrix) layer** | **Matrix weight [g/m²]** | **Backing layer/ Release liner** |
|---|---|---|---|---|
| 1 | 043LUMTDS | 15% Lumateperone | 50 | Scotchpak 9738/ Primeliner 78HL |
| | | 85% Duro-Tak^{®} 87-4098 | | |
| | | | | |
| 2 | 044LUMTDS | 15% Lumateperone | 50 | Scotchpak 9738/ Primeliner 78HL |
| | | 85% Duro-Tak^{®} 387-2516 | | |
| 3 | 058LUMTDS | 15% Lumateperone | 50 | Scotchpak 9738/ Primeliner 78HL |
| | | 84.95% Duro-Tak^{®} 387-2516 | | |
| | | 0.05% Tocopherol | | |
| 4 | 045LUMTDS | 15% Lumateperone | 50 | Scotchpak 9738/ Primeliner 78HL |
| | | 84.90% Duro-Tak^{®} 387-2516 | | |
| | | 0.10% Tocopherol | | |
| 5 | 073LUMTDS | 15% Lumateperone | 50 | Scotchpak 9738/ Primeliner 78HL |
| | | 84.95% Duro-Tak^{®} 387-2516 | | |
| | | 0.05% ascorbyl palmitate | | |
| 6 | 046LUMTDS | 15% Lumateperone | 50 | Scotchpak 9738/ Primeliner 78HL |
| | | 84.90% Duro-Tak^{®} 387-2516 | | |
| | | 0.10% ascorbyl palmitate | | |
| 7 | 047LUMTDS | 15% Lumateperone | 50 | Scotchpak 9738/ Primeliner 78HL |
| | | 84.90% Duro-Tak^{®} 387-2516 | | |
| | | 0.10% butylhydroxytoluene (BHT) | | |
| | | | | |

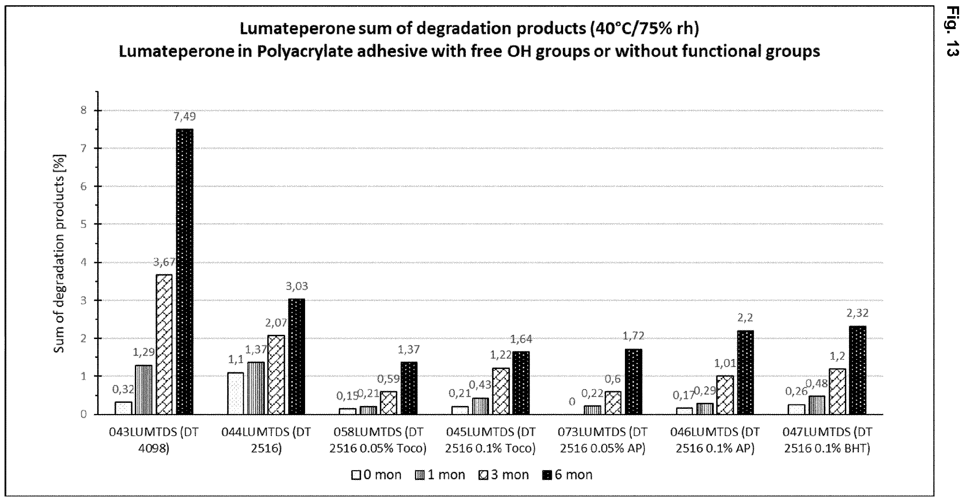

The formation of degradation products was monitored for 6 months at 40°C/75% RH. The results are shown in **Fig. 13****.**

It can be seen that the use of polyacrylate with free hydroxy groups leads to less degradation products of lumateperone free base than polyacrylate without functional groups (cf. sample 043LUMTDS vs 044LUMTDS). The use of antioxidants, e.g. tocopherol from about 0.05 to about 0.1% (058LUMTDS, 045LUMTDS), ascorbyl palmitate from about 0.05 to about 0.1% (073LUMTDS, 046LUMTDS), and butylhydroxytoluene (BHT) of about 0.1 % (047LUMTDS) shows a stabilizing effect and thus a reduction in the formation of degradation products. The use of tocopherol and ascorbyl palmitate as stabilizing antioxidants is preferred over the use of butylhydroxytoluene (BHT).

### Example 2: Bilayer formulation with adhesive layer (second aspect of the invention)

### a) Preparation

An active ingredient containing layer (active matrix layer) containing the active ingredient lumateperone free base, a polyacrylate adhesive with free hydroxy groups (Duro-Tak^{®} 387-2516), and, optionally, further excipients (including the antioxidant tocopherol) was coated on an intermediate release liner (Primeliner^{™} 78HL). The solvents were removed in a drying oven. The active ingredient containing layer was then laminated with an aluminized Scotchpak^{™} 9738 PET film as a backing layer and stored until further processing.

Separately, an adhesive layer initially free of the active ingredient lumateperone free base and containing an adhesive polymer and, optionally, further excipients, was coated on a release liner (Primeliner^{™} 78HL). The solvents were removed in a drying oven.

The intermediate release liner of the active ingredient containing layer was removed and the adhesive layer initially free of active ingredient was laminated with the active ingredient containing layer, forming a layered system consisting of: backing layer, active ingredient containing matrix layer, initially drug-free adhesive layer and release liner (protective film).

The resulting laminates are shown in the following **Table 4.**

**Table 4**

| **No.** | **Batch** | **Composition of the (dried) active ingredient containing (matrix) layer** | **Matrix weight [g/m²]** | |
|---|---|---|---|---|
| 1 | 033LUMTDS | 15% Lumateperone | 50 | |
| | | 85% Duro-Tak^{®} 387-2516 | | |
| 2 | 033_034LUMTDS | 15% Lumateperone | 50 | |
| | | 85% Duro-Tak^{®} 387-2516 | | |
| 3 | 033_035LUMTDS | 15% Lumateperone | 50 | |
| | | 85% Duro-Tak^{®} 387-2516 | | |
| 4 | 033_036LUMTDS | 15% Lumateperone | 50 | |
| | | 85% Duro-Tak^{®} 387-2516 | | |
| | | | | |
| 5 | 065LUMTDS | 15% Lumateperone | 50 | |
| | | 0.05% Tocopherol | | |
| | | 84.95% Duro-Tak^{®} 387-2516 | | |
| 6 | 065_066LUMTDS | 15% Lumateperone | 50 | |
| | | 0.05% Tocopherol | | |
| | | 84.95% Duro-Tak^{®} 387-2516 | | |
| 7 | 065_067LUMTDS | 15% Lumateperone | 50 | |
| | | 0.05% Tocopherol | | |
| | | 84.95% Duro-Tak^{®} 387-2516 | | |

| **No.** | **Batch** | **Composition of the (dried) adhesive layer** | **Weight adhesive layer [g/m²]** | **Backing layer/ Release liner** |
|---|---|---|---|---|
| 1 | 033LUMTDS | - | - | Scotchpak 9738 |
| | | | | Primeliner 78HL |
| 2 | 033_034LUMTDS | 100% Duro-Tak^{®} 87-6173 | 30 | Scotchpak 9738 |
| | | | | Primeliner 78HL |
| 3 | 033_035LUMTDS | 30% Duro-Tak^{®} 87-626A | 30 | Scotchpak 9738 |
| | | 20% Duro-Tak^{®} 87-6900 | | Primeliner 78HL |
| | | 30% Indopol H-1900 | | |
| | | 20% Indopol H-18000 | | |
| 4 | 033_036LUMTDS | 100% Duro-Tak^{®} 387-2051 | 30 | Scotchpak 9738 |
| | | | | Primeliner 78HL |
| | | | | |
| 5 | 065LUMTDS | - | - | Scotchpak 9738 |
| | | | | Primeliner 78HL |
| 6 | 065_066LUMTDS | 100% Duro-Tak^{®} 87-6911 | 30 | Scotchpak 9738 |
| | | | | Primeliner 78HL |
| 7 | 065_067LUMTDS | 98% Duro-Tak^{®} 387-2516 | 30 | Scotchpak 9738 |
| | | 2% Miglyol^{®} 812 | | Primeliner 78HL |

### b) Adhesive strength

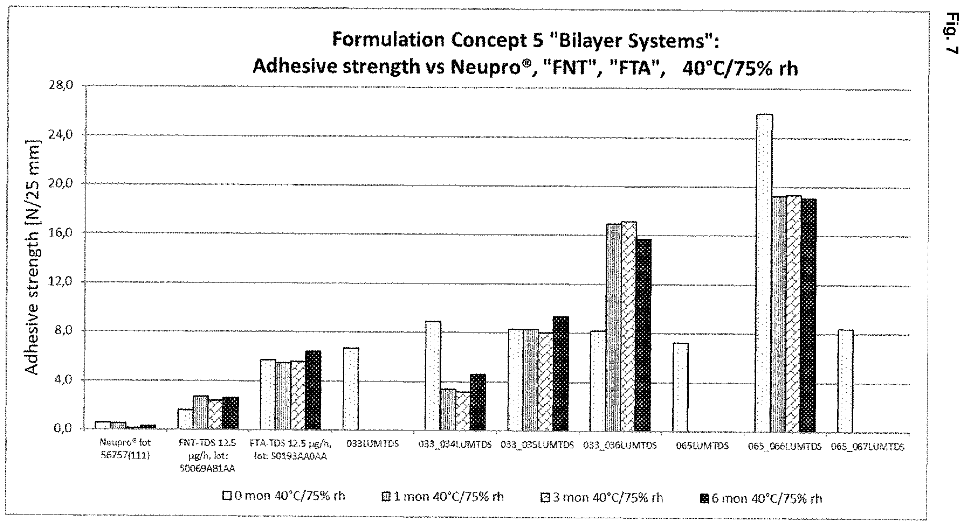

The adhesive strength was measured after 0 months, 1 months, 3 months and 6 months at 40°C/75% RH for some of the transdermal therapeutic systems prepared in Example 2. The results are shown in **Fig. 7****.**

From **Fig. 7****,** it can be taken that the adhesive strength of an active ingredient containing layer based on polyacrylate with free hydroxy groups (Duro-Tak^{®} 387-2516) can be increased with the aid of an (initially) active ingredient-free adhesive layer based on polyisobutylene (Duro-Tak^{®} 87-626A & 6900) and polybutene (Indopol H-1900 and/or H-18000) (cf. 033LUMTDS vs 033_035LUMTDS). It can further be increased when using an adhesive layer based on polyacrylate with free carboxylic acid groups (Duro-Tak^{®} 387-2051) (cf. 033LUMTDS vs 033_036LUMTDS).

The adhesive strength is slightly increased when using an adhesive layer based on polyacrylate with free hydroxy groups (Duro-Tak^{®} 387-2516) with a small amount of about 2 % Miglyol^{®} 812 (cf. 065LUMTDS vs 065_067LUMTDS).

The adhesive strength is noticeably increased when using an adhesive layer based on a styrene-butadiene-styrene copolymer (Duro-Tak^{®} 87-6911) (cf. 065LUMTDS vs 065_066LUMTDS).

The adhesive strength is constantly high during storage over 6 months after an initial decrease after 1 month of storage at 40°C/75% RH.

Adhesive layers based on polyisobutylene/polybutene (033_035LUMTDS), or polyacrylate with free carboxylic acid groups (065_067LUMTDS) do not show any relevant decrease in adhesive strength during storage over 6 months at 40°C/75% RH.

### c) Tack

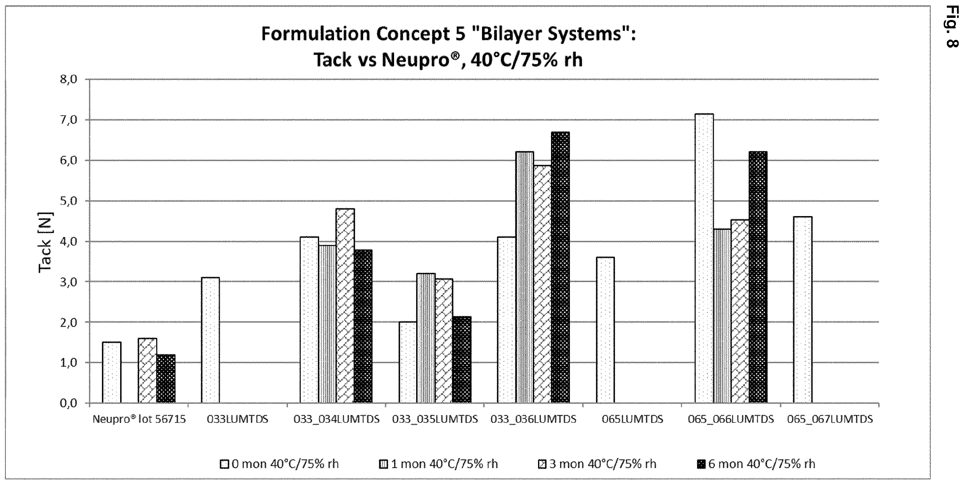

The tack was measured after 0 months, 1 months, 3 months and 6 months at 40°C/75% RH for some of the transdermal therapeutic systems prepared in Example 2. The results are shown in **Fig. 8****.**

From **Fig. 8****,** it can be taken that the tack of the active ingredient containing layer based on polyacrylate with free hydroxy groups (033LUMTDS; 065LUMTDS) can be significantly increased with the aid of an adhesive layer based on a polyacrylate with free carboxylic acid groups (Duro-Tak^{®} 387-2051) (cf. 033LUMTDS vs 033_036LUMTDS), or a styrene-butadiene-styrene copolymer (Duro-Tak^{®} 87-6911) (cf. 065LUMTDS vs. 065_066LUMTDS).

A certain increase can also be achieved by using an (initially) active ingredient-free adhesive layer based on polyacrylate with free hydroxy groups (Duro-Tak^{®} 387-2516) and adding a small amount of about 2% Miglyol^{®} 812 (cf. 065LUMTDS vs. 065_067LUMTDS).

There is no relevant decrease of tack during storage over 6 months at 40°C/75% RH.

### d) Separation force

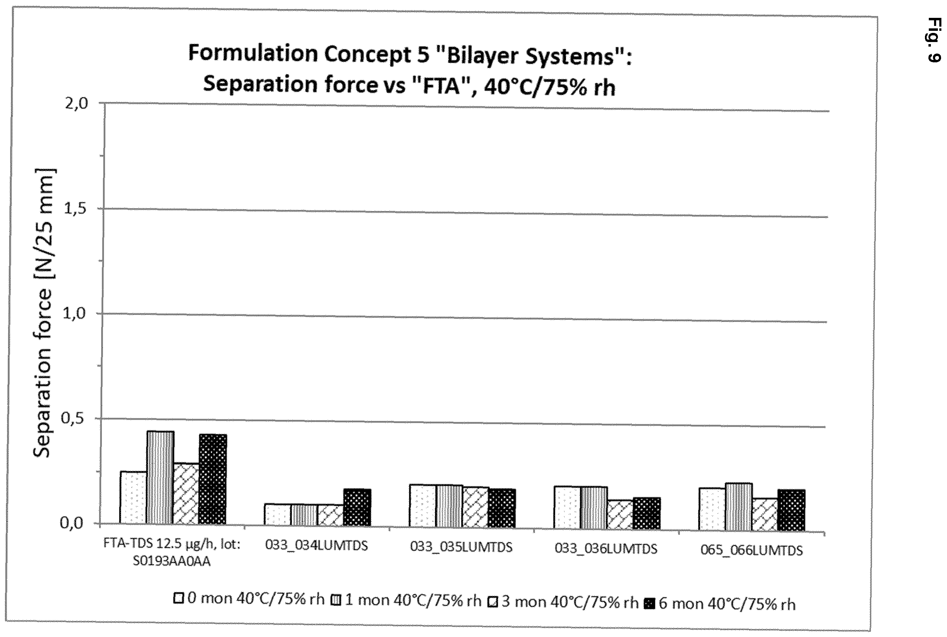

The separation force was measured after 0 months, 1 months, 3 months and 6 months at 40°C/75% RH for some of the transdermal therapeutic systems prepared in Example 2. The results are shown in **Fig. 9****.**

From **Fig. 9****,** it can be taken that all of the tested systems (033_034LUMTDS; 033_035LUMTDS; 033_036LUMTDS; 065_066LUMTDS) exhibit only low separation forces and, moreover, show no relevant increase in separation force during storage over 6 months at 40°C/75% RH.

### e) Skin permeation

### aa)Preliminary remarks

In order to realize a small patch size, the cumulative permeated amount per 24 h must be sufficiently high. An amount of about 1.8 mg lumateperone must be provided transdermally at an oral bioavailability of about 4.4% per day. For a patch size of about 20 cm², a flux of at least 3.75 µg/cm²*h must be achieved (3.75 µg/cm²*h x 20 cm² x 24 = 1,800 µg/24 h = 1.8 mg/24 h), which corresponds to a transdermally delivered amount of 90 µg per day and cm². Ideally, this flux is achieved without the use of permeation enhancers.

### bb)Skin permeation through human skin model (HSE)

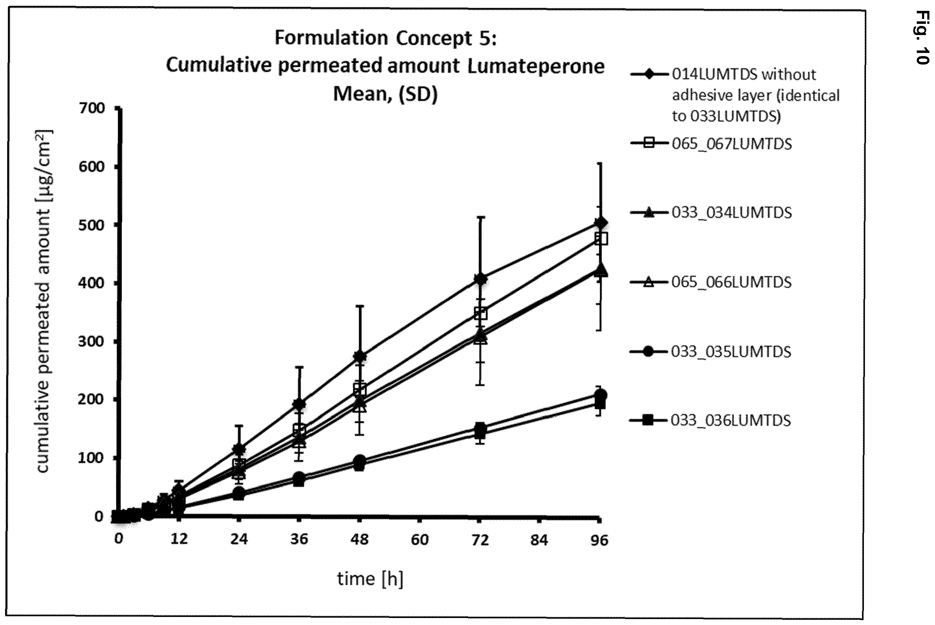

The permeation of selected samples prepared in Example 2 was measured on human skin over 96 hours in order to determine the cumulatively permeated amount of lumateperone per cm². The results are depicted in **Fig. 10****.**

The results show that when using an adhesive layer, the time period wherein permeation is constant can be increased from about 2 days to about 3 days compared to the sample without adhesive layer (014LUMTDS). At the same time, however, permeation is reduced compared to the sample without adhesive layer (014LUMTDS).

The samples show the following permeation effect on human skin model (HSE) ranging from high to low:
no adhesive layer (014LUMTDS) > 98% Duro-Tak^{®} 387-2516, 2% Miglyol^{®} (065_067LUMTDS) > 100% Duro-Tak^{®} 87-6173 (33_034LUMTDS) ~ 100% Duro-Tak^{®} 87-6911 (065_066LUMTDS) >> 30/20% Duro-Tak^{®} 87-626A/-6900, 30/20% Indopol H-1900/-18000 (033_035LUMTDS) ~ 100% Duro-Tak^{®} 87-6911 (033_036LUMTDS).

Adhesive layers based on a styrene-butadiene rubber (033_034LUMTDS) or a styrene-butadiene-styrene copolymer (065_066LUMTDS), or based on a polyacrylate with free hydroxy groups and a small amount of Miglyol^{®} 812 (065_067LUMTDS) prolog the time period, wherein permeation is constant, and allow a small patch size of about 20 cm².

### Example 3: Bilayer formulation with adhesive layer and drug-permeable membrane (third aspect of the invention)

### a) Preparation

An active ingredient containing layer containing the active ingredient lumateperone free base, a polyacrylate adhesive with free hydroxy groups (Duro-Tak^{®} 387-2516), and, optionally, further excipients (such as the antioxidant tocopherol) was coated on an intermediate release liner (Primeliner^{™} 78HL). The solvents were removed in a drying oven. The active ingredient containing layer was then laminated with an aluminized PET film (Scotchpak^{™} 9738) as a backing layer and stored until further processing.

Separately, an adhesive layer free of the active ingredient was coated on a release liner (Primeliner^{™} 78HL). The solvents were removed in a drying oven. The adhesive layer was then laminated with a drug-permeable membrane layer.

The intermediate release liner of the active ingredient containing layer was removed and the exposed active ingredient containing layer was laminated to the membrane side of the adhesive layer, forming a layered system including: backing layer, active ingredient containing layer, drug-permeable membrane, initially drug-free adhesive layer and release liner.

The resulting laminates are shown in the following **Table 5:**

**Table 5**

| **No.** | **Batch** | **Composition of the (dried) active ingredient containing (matrix) layer** | **Weight matrix [g/m²]** | **Membrane layer** |
|---|---|---|---|---|
| 1 | 065_067LUMTDS | 15% Lumateperone | 50 | - |
| | | 0.05% Tocopherol | | |
| | | 84.95% Duro-Tak^{®} 387-2516 | | |
| 2 | 065_068LUMTDS | 15% Lumateperone | 50 | PE |
| | | 0.05% Tocopherol | | Cotran^{™} 9719 |
| | | 84.95% Duro-Tak^{®} 387-2516 | | |
| 3 | 065_069LUMTDS | 15% Lumateperone | 50 | EVA |
| | | 0.05% Tocopherol | | Cotran^{™} 9728 |
| | | 84.95% Duro-Tak^{®} 387-2516 | | |
| 4 | 065_070LUMTDS | 15% Lumateperone | 50 | Porous PE |
| | | 0.05% Tocopherol | | Solupor^{™} |
| | | 84.95% Duro-Tak^{®} 387-2516 | | 10P05A |
| 5 | 065_071LUMTDS | 15% Lumateperone | 50 | Porous PP |
| | | 0.05% Tocopherol | | Celgard^{™} 2400 |
| | | 84.95% Duro-Tak^{®} 387-2516 | | |

| **No.** | **Composition of the (dried) adhesive layer** | | **Weight adhesive layer [g/m²]** | **Backing layer/ Release liner** |
|---|---|---|---|---|
| 1 | 98% Duro-Tak^{®} 387-2516 | | 30 | Scotchpak 9738 |
| | 2% Miglyol^{®} 812 | | | Primeliner 78HL |
| 2 | 98% Duro-Tak^{®} 387-2516 | | 30 | Scotchpak 9738 |
| | 2% Miglyol^{®} 812 | | | Primeliner 78HL |
| 3 | 98% Duro-Tak^{®} 387-2516 | | 30 | Scotchpak 9738 |
| | 2% Miglyol^{®} 812 | | | Primeliner 78HL |
| 4 | 98% Duro-Tak^{®} 387-2516 | | 30 | Scotchpak 9738 |
| | 2% Miglyol^{®} 812 | | | Primeliner 78HL |
| 5 | 98% Duro-Tak^{®} 387-2516 | | 30 | Scotchpak 9738 |
| | 2% Miglyol^{®} 812 | | | Primeliner 78HL |

### a) Skin permeation

### aa)Preliminary remarks

In order to realize a small patch size, the cumulative permeated amount per 24 h must be sufficiently high. An amount of about 1.8 mg lumateperone must be provided transdermally at an oral bioavailability of about 4.4% per day. For a patch size of about 20 cm², a flux of at least 3.75 µg/cm²*h must be achieved (3.75 µg/cm²*h x 20 cm² x 24 = 1,800 µg/24 h = 1.8 mg/24 h), which corresponds to a transdermally delivered amount of 90 µg per day and cm². Ideally, this flux is achieved without the use of permeation enhancers.

### bb)Permeation on human skin model (HSE)

The permeation of selected samples prepared in Example 3 was measured on human skin in order to determine the cumulatively permeated amount of lumateperone per cm² over a period of 96 hours.

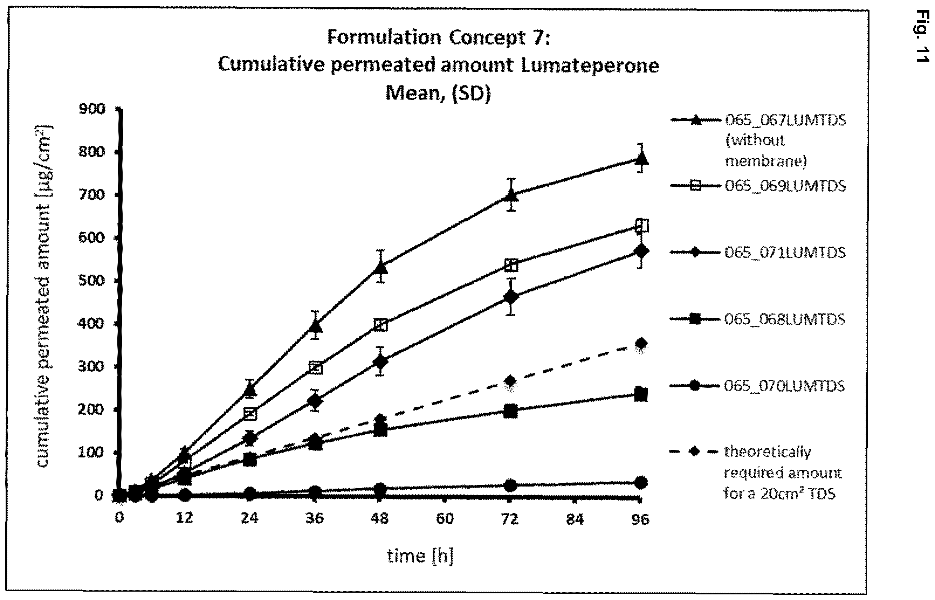

The results are depicted in **Fig. 11****.**

The samples show the following prolongation of the constant permeation time on human skin model (HSE) from low to high compared to the reference formulation with no membrane based on the type of membrane:
no membrane (065_067LUMTDS): 2 days <
polyethylene vinyl acetate (EVA) membrane (065_069LUMTDS): 2 days <
porous polypropylene (PP) membrane (065_071LUMTDS): 3 days <<
coherent polyethylene (PE) membrane (065_068LUMTDS): 4 days <<
porous polyethylene (PE) membrane (065_070LUMTDS): 4 days.

Using a porous polypropylene membrane (065_071LUMTDS) or EVA membrane (065_069LUMTDS), the permeation is only slightly reduced, whereas the constant permeation time is only extended from about 2 days to about 3 days for the porous polypropylene membrane.

A coherent polyethylene membrane (065_068LUMTDS) provides a strong reduction of permeation and thus an extension of the constant permeation time from about 2 days to 4 days. A porous polyethylene membrane (065_070LUMTDS) reduces permeation significantly.

### Example 4: Monolayer formulation based on a silicone polymer (first aspect of the invention)

### a) Preparation

An active ingredient layer containing the active ingredient lumateperone free base, a silicone adhesive, and, optionally, further excipients (Tocopherol), was coated on a protective film (Primeliner^{™} 78HL or Scotchpak^{™} 9709). The solvents were removed in a drying oven. The active layer was then laminated with a backing layer (Hostaphan^{™} MND 12 Med or Scotchpak^{™} 9738).

The resulting laminates are shown in the following **Table 6.**

**Table 6**

| **No.** | **Batch** | **Composition of the (dried) active ingredient (matrix) layer** | **Matrix weight [g/m²]** | **Backing layer/ Release liner** |
|---|---|---|---|---|
| 1 | 010LUMTDS | 12.6% Lumateperone | 50 | Hostaphan MN12/ |
| | | 87.4% BIO-PSA 7-4302 | | Scotchpak 1022 |
| 2 | 013LUMTDS | 15% Lumateperone | 50 | Scotchpak 9738/ |
| | | 85% BIO-PSA 7-4302 | | Scotchpak 9709 |
| 3 | 072LUMTDS | 15% Lumateperone | 50 | Scotchpak 9738/ |
| | | 0.05% Tocopherol | | Scotchpak 9709 |
| | | 84,95% BIO-PSA 7-4202 | | |

### b) Crystallization and phase stability

Some of the samples prepared in Example 5 were analyzed for the occurrence of crystallization and the formation of a biphasic system after being stored at 40°C/75% RH. The results are shown in the following **Table 7.**

**Table 7**

| **No.** | **Batch** | **Storage conditions** | **Crystallization visible?** | **Biphasic system visible (using a microscope)?** |
|---|---|---|---|---|
| 1 | 010LUMTDS | 6 months at rt | no | yes |
| 2 | 010LUMTDS | 12 months at 40°C/75% RH | no | yes |
| 3 | 013LUMTDS | 6 months at 40°C/75% RH | no | yes |

| | | | | |
|---|---|---|---|---|
| rt = room temperature; RH = relative humidity | | | | |

### c) Permeation

### aa)Preliminary remarks

In order to realize a small patch size, the cumulative permeated amount per 24 h must be sufficiently high. An amount of 1.8 mg lumateperone must be provided transdermally at an oral bioavailability of about 4.4% per day. For a patch size of about 20 cm², a flux of at least 3.75 µg/cm²*h must be achieved (3.75 µg/cm²*h x 20 cm² x 24 = 1,800 µg/24 h = 1.8 mg/24 h), which corresponds to a transdermally delivered amount of 90 µg per day and cm². Ideally, this flux is achieved without the use of permeation enhancers.

### bb)Permeation on human skin (HSE)

The permeation of a selected sample prepared in Example 4 (batch no. 013LUMTDS) was measured on human skin model (HSE) over a period of 96 hours in order to determine the cumulatively permeated amount of lumateperone µg/cm².

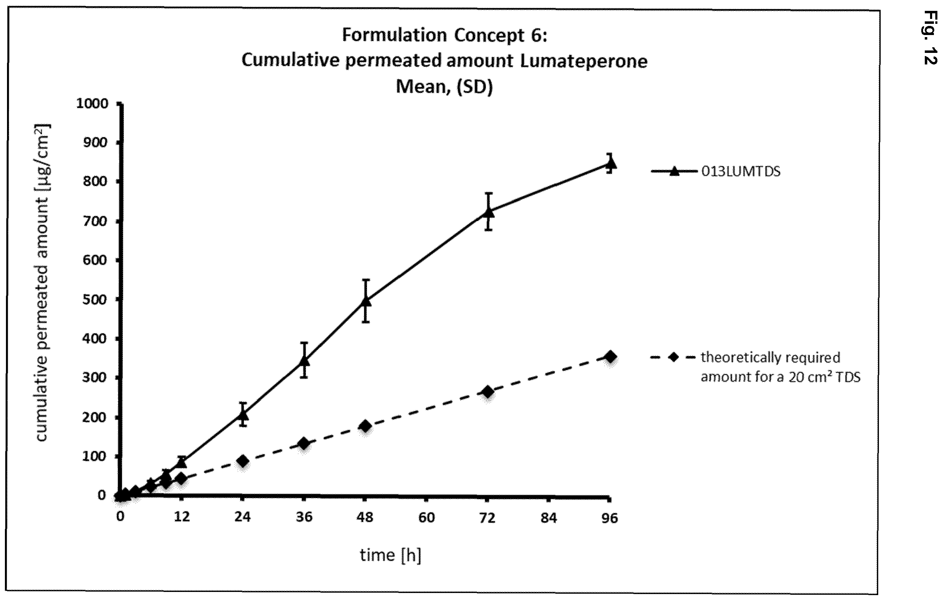

The results are depicted in **Fig. 12****.**

The dashed line in **Fig. 12** indicates the theoretically required amount of lumateperone for a 20 cm² TDS. The sample shows on human skin model (HSE) a high permeation exceeding the theoretically required amount for a 20 cm² TDS without the need of using a permeation enhancer.

## Claims

1. Transdermal therapeutic system for administering the active ingredient lumateperone through the skin of a patient comprising the following layers:
a) a backing layer and
b) at least one active ingredient containing layer, comprising the active ingredient lumateperone in free base or salt form embedded in a polymer matrix,
**characterized in that**
the polymer matrix comprises a polymer selected from the group consisting of (1) a polyacrylate, preferably a polyacrylate containing free hydroxy groups, (2) a mixture of a polyacrylate and a silicone polymer, and (3) a silicone polymer.

2. Transdermal therapeutic system according to claim 1, comprising the active ingredient lumateperone in free base form.

3. Transdermal therapeutic system according to claim 1 or 2, **characterized in that** the active ingredient containing layer comprises lumateperone in an amount in the range from 5 to 20 wt-%, based on the overall weight of the active ingredient containing layer.

4. Transdermal therapeutic system according to any one of the preceding claims, **characterized in that** the active ingredient containing layer further comprises one or more pharmaceutically acceptable excipients selected from the group consisting of antioxidants, permeation enhancers, plasticizers, tackifiers, crystallization inhibitors and cohesion increasing substances.

5. Transdermal therapeutic system according to any one of the preceding claims, **characterized in that** the active ingredient containing layer further comprises substances that trigger the *in situ* release of lumateperone free base in case the active ingredient is used in a salt form.

6. Transdermal therapeutic system according to any one of the preceding claims, **characterized in that** the active ingredient containing layer is the skin-contacting layer of the system.

7. Transdermal therapeutic system according to any one of the preceding claims, **characterized in that** it further comprises
c) an adhesive layer on the active ingredient containing layer,
wherein the adhesive layer comprises at least one adhesive polymer,
wherein the adhesive layer is initially free of the active ingredient,
and wherein the adhesive layer is the skin-contacting layer of the system.

8. Transdermal therapeutic system according to claim 7, **characterized in that** at least one adhesive polymer of the adhesive layer is selected from the group consisting of acrylate polymers and/or copolymers, styrene-butadiene-styrene (SBS) copolymers, polyisobutylene, preferably selected from the group consisting of a polyacrylate with free hydroxy groups and styrene-butadiene-styrene (SBS) copolymers.

9. Transdermal therapeutic system according to claim 7 or 8, **characterized in that** the adhesive layer consists of the at least one adhesive polymer, or further comprises one or more pharmaceutically acceptable excipients selected from the group consisting of permeation enhancers, plasticizers, softeners, cohesion increasing substances, antioxidants.

10. Transdermal therapeutic system according to any one of claims 7 to 9, **characterized in that** it further comprising
d) a membrane layer between the active ingredient containing layer and the adhesive layer,
wherein the membrane layer comprises a membrane polymer that is permeable for the active ingredient and controls the release of the active ingredient.

11. Transdermal therapeutic system according to claim 10, **characterized in that** the membrane polymer is a polyolefin selected from the group consisting of polypropylene, polyethylene, and of polyethylene vinyl acetate.

12. Transdermal therapeutic system according to any one of the preceding claims, **characterized in that** it further comprises
e) a release liner to protect the skin-contacting layer before use of the system.

13. Transdermal therapeutic system according to any one of the preceding claims, **characterized in that** the permeation is constant over a period of at least 48 hours, preferably at least 72 hours; and/or the flux is at least 3.75 µg/cm²*h; and/or the daily dosage delivered is at least 1.5 mg/day, preferably 1.8 mg/day; and/or the size of the transdermal therapeutic system is in the range from 4 to 40 cm².

14. Transdermal therapeutic system according to any one of the preceding claims, **characterized in that** it does not comprise a permeation enhancer.

15. A method for preparing a transdermal therapeutic system according to any one of the preceding claims, comprising the following steps:
(a) preparing an active ingredient containing layer composition,
(b) coating the composition prepared in step (a) onto a release liner (i),
(c) drying the component prepared in step (b), and
(d) laminating a backing layer onto the active ingredient layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa;*
or optionally comprising the following steps in addition to steps (a)-(d):
(j) laminating a membrane layer suitable to control the release of the active ingredient onto the active ingredient containing layer of the component prepared in accordance with step (h),
(k) performing steps (e) to (g),
(l) laminating the adhesive layer of the component prepared in step (k) onto the membrane layer of the component prepared in step (j), or *vice versa.*

16. Use of a polymer selected from the group consisting of (1) a polyacrylate, preferably a polyacrylate containing free hydroxy groups, (2) a mixture of a polyacrylate and a silicone polymer, and (3) a silicone polymer for stabilizing lumateperone free base in a transdermal therapeutic system or reducing decomposition of lumateperone free base in a transdermal therapeutic system, preferably in a transdermal therapeutic system as defined in any one of claims 1 to 14.

17. Use of an antioxidant, preferably ascorbic acid or tocopherol, in a transdermal therapeutic system as defined in any one of claims 1 to 14 for reducing, preferably inhibiting the formation of N-nitrosamines in the system.

18. Transdermal therapeutic system according to any of claims 1 to 14 for use in a method for treating a disease selected from the group consisting of major depressive disorder (MDD) and adjunctive mixed features associated therewith, bipolar depression I or II and schizophrenia.

19. Lumateperone for use in a method for treating a disease selected from the group consisting of major depressive disorder (MDD) and adjunctive mixed features associated therewith, bipolar depression I or II and schizophrenia, wherein lumateperone is administered in free base or salt form through the skin of a patient by means of the transdermal therapeutic system as defined in any one of claims 1 to 14.
